# EUROPEAN PATENT SPECIFICATION

(11) **EP 0 363 415 B1**
(45) Date of publication and mention of the grant of the patent: **15.10.2008**
(21) Application number: 88905083.7
(22) Date of filing: 04.05.1988
(51) Int. Cl.: A61K 31/55, C07D 491/06, C07D 487/06, A61P 25/28

(54) **COMPOUNDS FOR THE TREATMENT OF ALZHEIMER'S DISEASE**
MITTEL ZUR BEHANDLUNG DER ALZHEIMER-KRANKHEIT
COMPOSES PERMETTANT LE TRAITEMENT DE LA MALADIE D'ALZHEIMER

(30) Priority: 04.05.1987 US 46522
(43) Date of publication of application: 18.04.1990
(60) Divisional application: 07000875.0
(73) Proprietor: Davis, Bonnie, Huntington, New York (US)
(72) Inventor: JOULLIE, Madeleine, Philadelphia, PA 19106 (US)
(74) Representative: Galloway, Peter David
(86) International application number: PCT/US1988/001542
(87) International publication number: WO 1988/008708

(56) References cited:
- GB-A- 2 039 892
- NL-A- 8 800 350
- US-A- 3 673 177
- ANTISEPTIC (INDIA), vol. 71, no. 1, 1974, pages 45-47; P.A. BHASKER: "Medical management of dementia"
- CHEMICAL ABSTRACTS, vol. 57, 1962, abstract no. 12560e, Columbus, Ohio, US; L. BUBEVA-IVANOVA: "Amaryllidaceae alkaloids. VI. The effect of several mineral acids on galanthamine", & BER. 95, 1348-53(1962)
- CHEMICAL ABSTRACTS, vol. 58, 1963, abstract no. 11411b, Columbus, Ohio, US; G. COMBES et al.: "Galanthaminone", & BULL. SOC. CHIM. FRANCE 1962, 1805-9
- CHEMICAL ABSTRACTS, vol. 61, 1964, abstract no. 14732f, Columbus, Ohio, US; S.M. LAIHO et al.: "Narcissamine. A quasi-racemic alkaloid", & J. AM. CHEM. SOC. 86(20), 4434-8(1964)
- CHEMICAL ABSTRACTS, vol. 61, 1964, abstract no. 13357d, Columbus, Ohio, US; J. KOIZUMI et al.: "Galanthamine chemistry. V. Formation of hydroxyapogalanthamine from galanthaminone and the synthesis of its trimenthyl ether", & CHEM. PHARM. BULL. (TOKYO) 12(6), 696-705(1964)
- CHEMICAL ABSTRACTS, vol. 59, 1963, abstract no. 3716f, Columbus, Ohio, US; A. ABDUSAMATOV et al.: "Alkaloids from Ungernia taoshikorum and derivatives of alkaloids from U victoris", & DOKL. AKAD. NAUK. UZ. SSR 20(1), 18-21(1963)
- CHEMICAL ABSTRACTS, vol. 61, 1964, abstract no. 14727g, Columbus, Ohio, US; S. MINAMI et al.: "Galanthamine chemistry. VI. Synthesis of deoxydemethyllycoramine", & CHEM. PHARM. BULL. (TOKYO) 12(9), 1012-20(1964)
- CHEMICAL ABSTRACTS, vol. 74, 1971, abstract no. 112265z, Columbus, Ohio, US; T. KAMETANI et al.: "Syntheses of heterocyclic compounds. CCCLXXXI. Novel conversion of galanthamine into nivalidine under nonacidic conditions", & J. CHEM. SOC. C 1971, (3), 590-2
- CHEMICAL ABSTRACTS, vol. 78, 1973, page 507, abstract no. 111558t, Columbus, Ohio, US; T. KAMETANI et al.: "Syntheses of analgesics. XXXV. Syntheses of heterocyclic compounds. DVII. Synthesis of (+)-N-norgalanthamine", & J. HETEROCYCL. CHEM. 1973, 10(1), 35-7
- CHEMICAL ABSTRACTS, vol. 77, 1972, page 515, abstract no. 48690s, Columbus, Ohio, US; & JP-A-72 13 919 (GRELAN PHARMACEUTICAL CO., LTD) 26-04-1972
- CHEMICAL ABSTRACTS, vol. 77, 1972, page 526, abstract no. 62186p, Columbus, Ohio, US; T. KAMETANI et al.: "Syntheses of heterocyclic compounds. CDLXVI. Synthesis of narwedine-type enones by photochemical cyclization", & J. CHEM. SOC., PERKIN TRANS. 1 1972, (12), 1513-16
- CHEMICAL ABSTRACTS, vol. 88, 1978, page 612, abstract no. 7146c, Columbus, Ohio, US; A.G. SCHULTZ et al.: "Total synthesis of dl-lycoramine", & J. AM. CHEM. SOC. 1977, 99(24), 8065-7
- CHEMICAL ABSTRACTS, vol. 66, 1967, page 8772, abstract no. 93920a, Columbus, Ohio, US; M. CECCHINI: "Treatment of motor impairment following cerebral vascular disorders with galanthamine hydrobromide", & MINERVA MED. 57(83), 3385-6(1966)
- R.H.F. MANSKE: "The Alkaloids, Chemistry and Physiology", vol. VI, 1960, pages 338-343, and vol. XI, 1968, pages 348-352, Academic Press, New York, US
- THE LANCET, vol. 1, no. 8065, 25th March 1978, pages 659-660; A. COMFORT: "Cholinesterase inhibition in treatment of Alzheimer's dementia"
- J.A.M.A., vol. 238, no. 21, 21st November 1977, pages 2293-2294; A. BARAKA et al.: "Reversal of central anticholinergic syndrome by galanthamine"
- ZH. VYSSH. NERV. DEINT., vol. 26, 1976, pages 1091-1093; S.R. CHAPLYGINA et al.: "The action of cholinergic drugs in experimental amnesia"
- PHARMACOLOGY BIOCHEMISTRY & BEHAVIOR, vol. 26, no. 3, March 1987, pages 625-629, Pergamon Journals Ltd, US; M. BRUFANI et al.: "A long-lasting cholinesterase inhibitor affecting neural and behavioral processes"
- A. G. SCHULTZ, J. Amer. Chem. Soc. 99, 8065 (1977), see 9a on page 8066.
- CHEMICAL ABSTRACTS, Volume 73, No. 4, issued 27 July 1970 (Columbus, Ohio, USA) J. G. BHANDARKAR, "Structure and Biosynthesis of Chlidanthine". see page 392, column 1, Abstract No. 25709h, J. Chem. Soc. C 1970 (9), 1224-7. see (I)
- CHEMICAL ABSTRACTS, Vol. 51, No. 11 issued 10 June 1956 (Columbus, Ohio, USA) H. G. BOIT, "Amaryllidacae Alkaloids", The Abstract at 8120f, Chem. Ber. 89, 2462-5 (1956).
- CHEMICAL ABSTRACTS, Vol. 85. No. 21 issued 22 November 1976 (Columbus, Ohio USA) S. KOBAYASHI, "Alkaloids of the Amaryllidaceae". See page 577, Col. 1, Abstract No. 160383k, Chem. Pharm. Bull. 1976, 24(7) 1537-43.
- CHEMICAL ABSTRACTS, Vol. 95. No. 3 issued 20 July 1981 (Columbus, Ohio, USA) S. KOBAYASHI, "Isolation of O-Demethyl Coramine from Bulbs of Lycoris Radiata Herb". See page 728 column 1, Abstract No. 25351q Chem. Pharm. Bull. 1980, 28(11) 3433-6.
- CHEMICAL ABSTRACTS, Vol. 103, No. 1 issued 8 July 1957 (Columbus, Ohio, USA), LOSEV, "Treatment of Parkinsonianism with Metamisyl". See page 61, col. 2, the Abstract No. 625c Otkrytiya, Izobret. 1985 (13), 12.
- IRWIN R.L. ET AL: 'INHIBITION OF RAT BRAIN CHOLINESTERASE AFTER ADMINISTRATION OF THE DIMETHYLCARBAMATES OF DEOXYDEMETHYLLYCORAMINE, NEOSTIGMINE, OR PHYSOSTIGMINE' J. PHARM. EXP. THER. vol. 136, 1962, pages 20 - 25, XP008073358
- BORES G.M. ET AL DRUGS OF THE FUTURE vol. 21, no. 6, 1996, pages 627 - 636

## Description

The present invention is directed to galanthamine-analogues and their preparation and use for treatment of Alzheimer's disease.

United States Patent No. 4,663,318 issued on May 5, 1987 describes the use of galanthamine for the treatment of Alzheimer's disease and related dementias.

Published European Patent Application 236634 describes the use of galanthamine and certain analogues for the treatment of Alzheimer's disease and related dementias, such analogues have the formula: 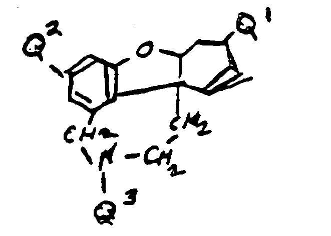 wherein Q¹ is methoxy, ethoxy, lower alkanoyloxy or oxy,

Q² is hydrogen, methoxy, ethoxy or lower alkanoyloxy and Q³ is straight or branched chain alkyl group, or cycloalkyl alkyl group, allyl or substituted lower alkyl phenyl and analogues thereof wherein hydrogen atoms ara replaced by chloro or fluoro atoms.

Without wishing to be bound by any theory, we now believe that the activity of galanthamine probably stems from the fact that a hydrogen bond between the hydroxyl group and the oxygen of the furan ring helps to stabilize its cyclohexene ring in a boat rather than a chair configuration:

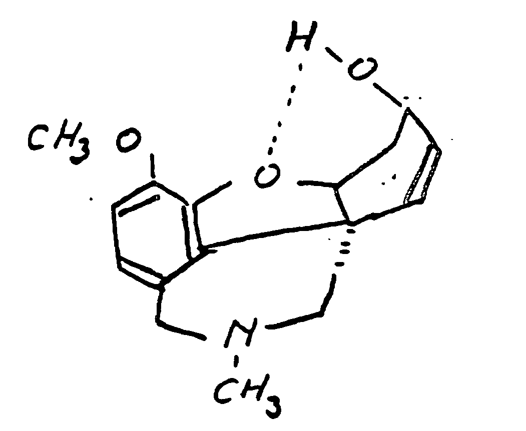

The literature shows the epigalanthamine wherein the hydroxy group is in the equatorial position so that hydrogen bond stabilization of structure is not possible has an anticholinesterase activity of only 10% of galanthamine.
(Chemical Abstracts Vol. 77 Abstract 109461s).

A number of galanthamine analogs occur naturally or have been obtained from natural products including the following compounds described in the text book The Alkaloids (edited by Manske published by Academic: New York, 1975, vol 15:
Narwedine:
(also referred to sometimes as galanthaminons)

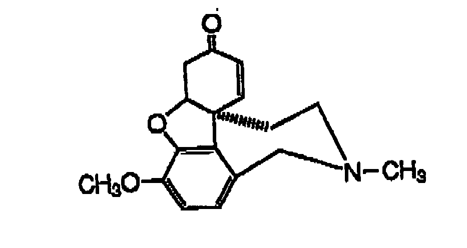

Narwedine has been reported to have weak anticurare activity at 3mg/kg (Chemical Abstracts Vol. 78 Abstract 131941r) and to have cholinergic effects on respiration and heart activity 50-80% lower than galanthamine (Chemical Abstracts Vol. 80 Abstract 103864r), Schmidt et al in Acta Biol Med Ger (7) 402-410 (1961) report that the anticholinesterase activity of Narwedene is less than 1% of that of galanthamine.
(-) N-demethyl galanthamine: 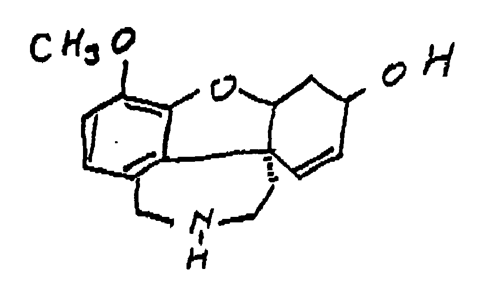 Galanthamine O methyl ether: 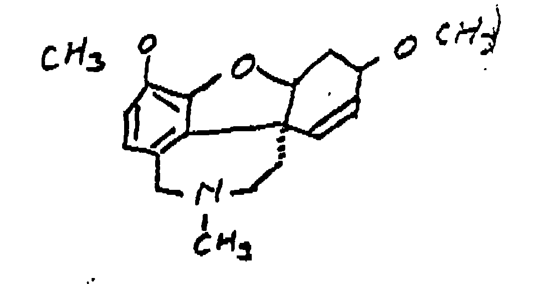 Childanthine: 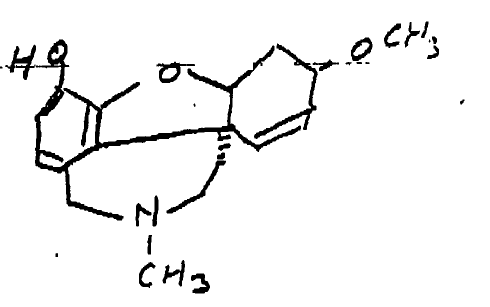 Lycoramine:

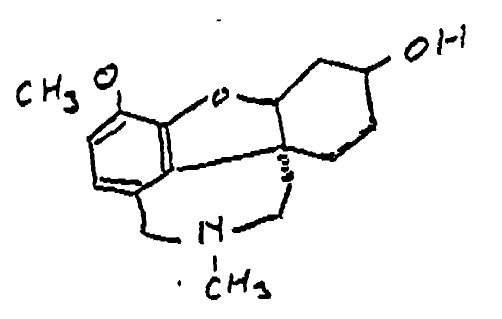

It has been reported that the concentration of lycoramine required to stimulate EEG - recorded activity is about five times that required for galanthamine (Chemical Abstracts Vol. 62 Abstract 15306a).
Deoxy lycoramine 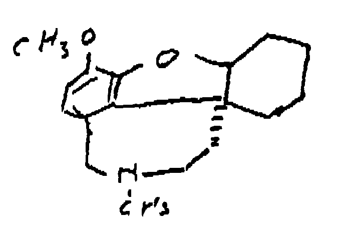 Habranthine: 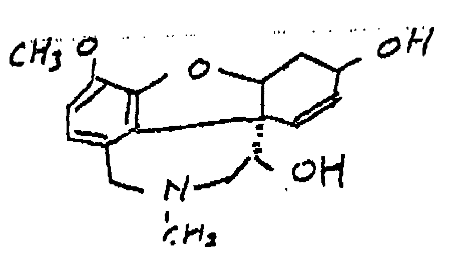 Anhydrogalanthamine: 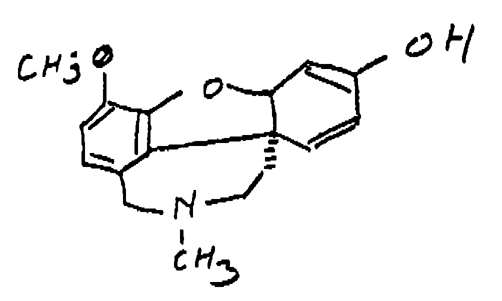 Anhydro-O-demethylgalanthamine: 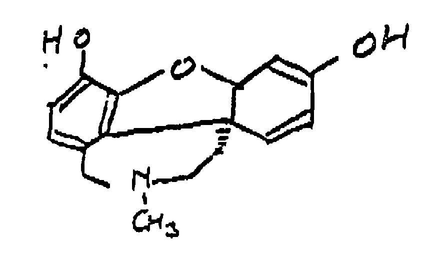 and N-mesityl and N-benzyl derivatives of galenthamine.

Reference is also made to compounds of the formulae:

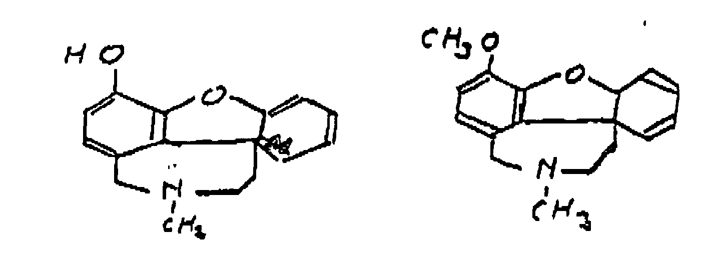

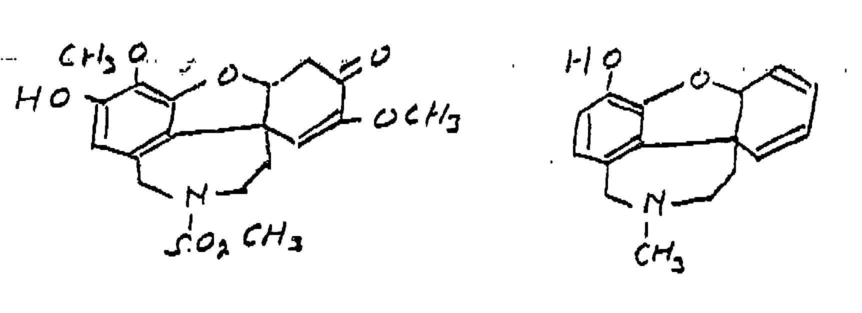

The latter compound has been reported to have activity similar to that of galanthamine (J Chem Soc (c) p 1043 (1971)

An earlier edition of this book had also referred to (+) N-demethyl dihydrogalanthamine of the formula:

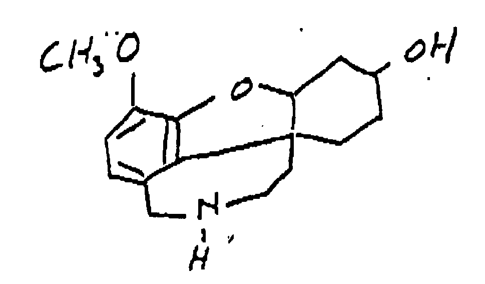

N-demethyl dihydrogalanthamine is described by Kametani et al in J. Heterocyclic Chemistry 1973 10(1) 35-7. The same paper also refers to galanthamine O, N diacetate Leucotamine 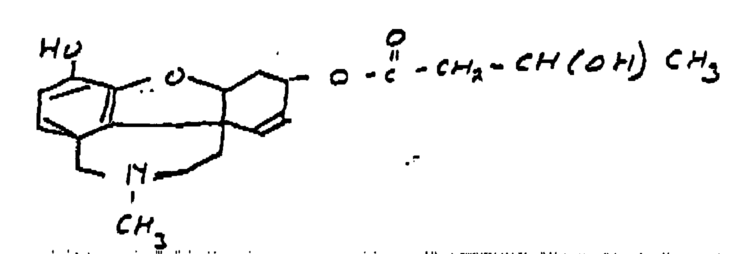 and its O-methyl and O-methyl acetic acid aster are described by Kobayashi et al in Chem. Pharm. Bull. 33 p 5258 (1985).

O-Demethyldihydrogalanthamine (also known as O-demethyl lycoramine) and O-demethyl galanthamine (sanguinine) are described by Kobayashi et al in Chem. Pharm. Bull. 28 3433-3436 (1980).

A bromo-narwedine of the formula: 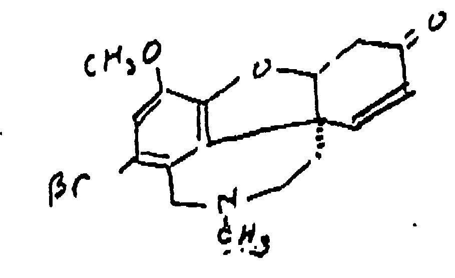 is described an an intermediate in the synthesis of galanthamine by Kametani at al in Cham, Comm, 1969 p, 425 and J. Chem. Soc, (C) (1969) 2602.

Chemical Abstracts 61 14727g Chem Pharm Bull 12 1012-20 (1964) describes the production of compounds of the formula: 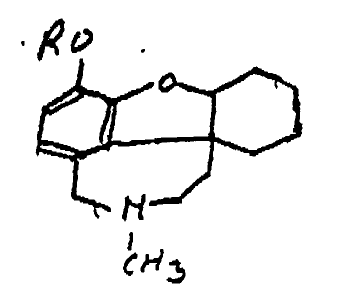 Wherein R may be hydrogen, methyl or ethyl.

Chemical Abstracts 81 638193 describes n.m,r, studies on dihydrogalanthamine and its acetic acid esters.

Shimizu et al in Heterocycles 1977 8 pages 277-82 (abstracted in Chemical Abstracts 88 136821t) describe certain narwedine derivatives used as intermediates having the following formulae:

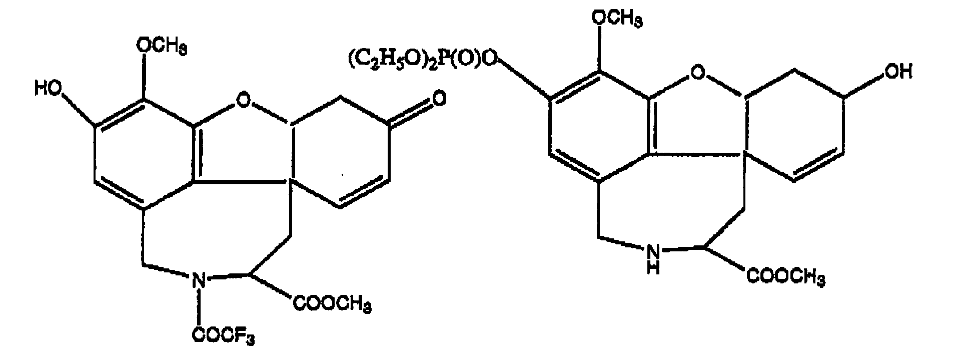

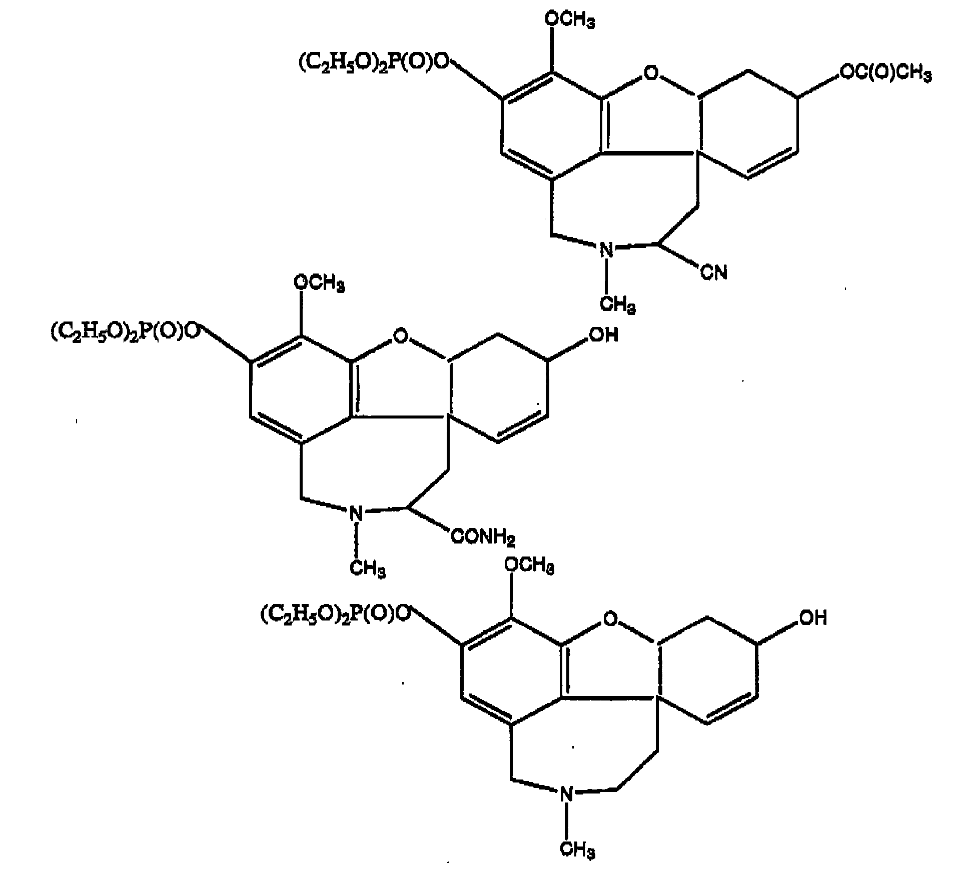

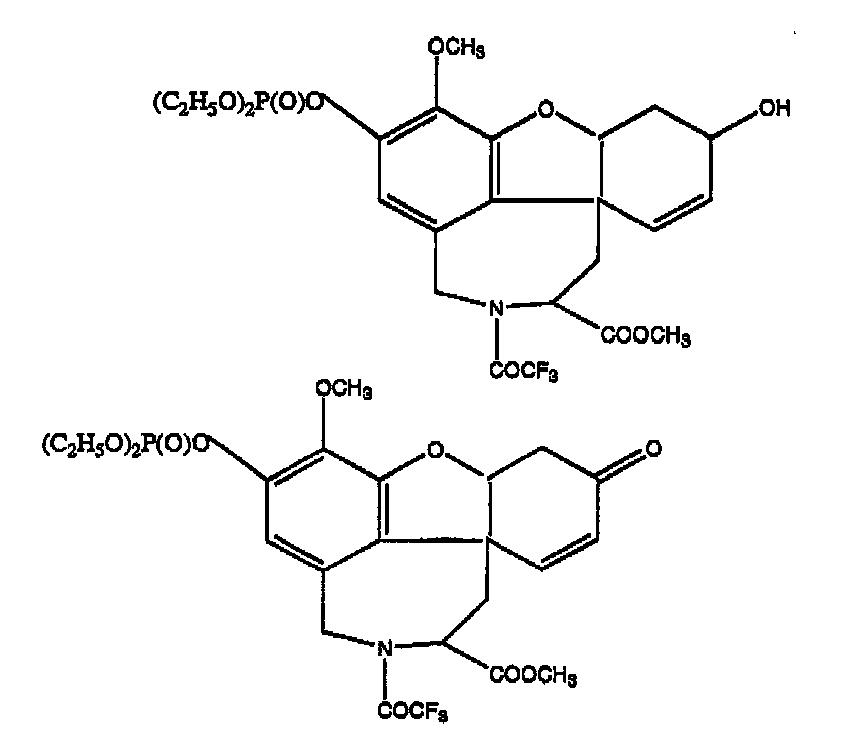

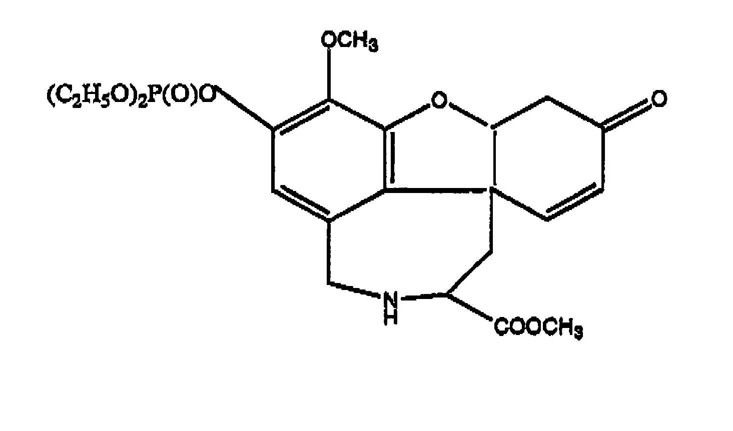

Irwin and Smith in Arch. Int. Pharmacodyn., 127:314-330 described the cholinesterase activity of galanthamine and some of its analogues including lycoramine acetate methiodide, neopin methiodide (both of which were ineffective) and deoxydemethyl lycoramine methiodide (which demonstrated good activity) and deoxylycoramine (which exhibited some activity). It was hypothesized that the presence of a free hydroxy group in the cyclohexane ring conferred activity on the molecule since the acetylation of this group led to a drop in activity.

In J. Pharm. Exp. Ther., 134:53 (1961). Irwin, Smith and Hein expanded on the work just described and reported that replacement of the hydroxyl group of deoxydemethyl lycoramine methiodide by a carbamate group resulted in useful pharmacological activity.

Subsequently, Irwin and Hine, J. Pharm. Exp, Ther., 136:20 (1962) described the activity of a number of carbamates including deoxydemethyl lycoramine carbamate on cholinesterase activity in rat brain.

Subsequently, Somers et al. in Neurology, 13:543 (1663) reported that some of Irwin's compounds were useful in treating myasthenia gravis.

### DETAILED DESCRIPTION OF THE INVENTION

From one aspect, the present invention-relates to the use of compounds of the formula I for the preparation of a medicament to treat Alzheimer's disease. 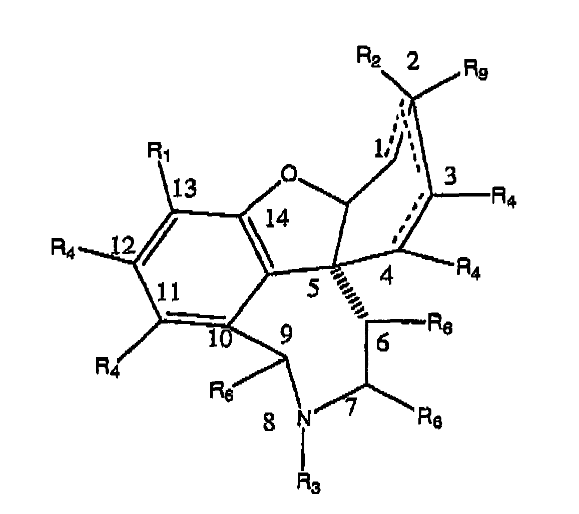 wherein the broken line represents an optionally present double bond in one or the two of the portions shown,

R₁ and R₂ are each selected independently from the group consisting of hydrogen, hydroxyl, cyano, sulfhydryl, alkoxy of 1-6 carbon atoms, aryloxy, R₅-substituted aryloxy, aralkoxy, R₅-substituted aralkoxy, aryloxymethyl, R₅-substituted aryloxymethyl, alkanoyloxy, hydroxy-substituted alkanoyloxy, benzoyloxy, R₅-substituted benzoyloxy, aryloxy carbonyl and R₅-substituted aryloxy carbonyl,

R₃ is hydrogen, straight or branched chain alkyl of 1-6 carbon atoms, cycloalkylmethyl, alkylphenyl,R₅-substituted alkylphenyl, heterocyclyl selected from α- or β-furyl, a or β-thienyl or thienyl, pyridyl, pyrazinyl and pyrimidyl, alkyl heterocyclyl or R'-substituted heterocyclyl, where heterocyclyl is as hereinabove defined and R' is alkyl or alkoxy
each R₄ is independently selected from hydrogen, hydroxyl, sulfhydryl, alkyl, aryl, aralkyl, alkoxy, aryloxy, alkaryloxy, nitro, amino, N-alkylamino, N-arylamino, N-alkarylamino, fluoro, chloro, bromo, and iodo ,
R₅ is selected from the same groups as R₄,
R₆ is hydrogen,
R₉ is hydrogen or is non-existant when the carbon in the 2 position is attached to a double bond
with the proviso that R₃ that is not methyl when R₁ is methoxy, R₂ is hydroxy and all R₄ are hydrogen and with the proviso that the compounds are not those wherein the hydroxy of galanthamine is replaced by methoxy, ethoxy, C₁₋₅ alkanoyloxy such as acetyloxy, the methoxy group is replaced by hydrogen, methoxy, ethoxy or lower alkanoyloxy of from 1 to 5 carbon atome such as acetoxy and the methyl group substituted on the nitrogen atom of galanthamie is replaced by other straight or branch chain C₁ - C₅ lower alkyl groups such as ethyl, cyclopropylmethyl or cyclobutylmethyl, allyl C₁-C₅ lower alkylphenyl or substituted lower C₁- C₅ alkylphenyl wherein the substituents are fluoro, chloro, bromo, lower alkoxy, hydroxy, nitro, amino, lower C₁-C₅ alkyl, or acyl amino of from 1 to 5 carbon atoms, heteroaryl lower C1 - C5 alkyl in which the heteroaryl group is thienyl, furyl, pyridyl, pyrrolyl, or pyrazinyl, or a cyano radical; or unsubstituted and halogen substituted benzoyl lower C₁ - C₅ alkyl in which the substituents are on the phenyl ring and compounds
wherein hydrogen atoms in the "core" structure have been replaced by fluoro or chloro groups or a pharmaceutically-acceptable acid addition salt thereof.

From a second aspect, the present invention provides for use of a compound of the following formula la for preparation of a medicament for treatment of Alzheimer's disease: 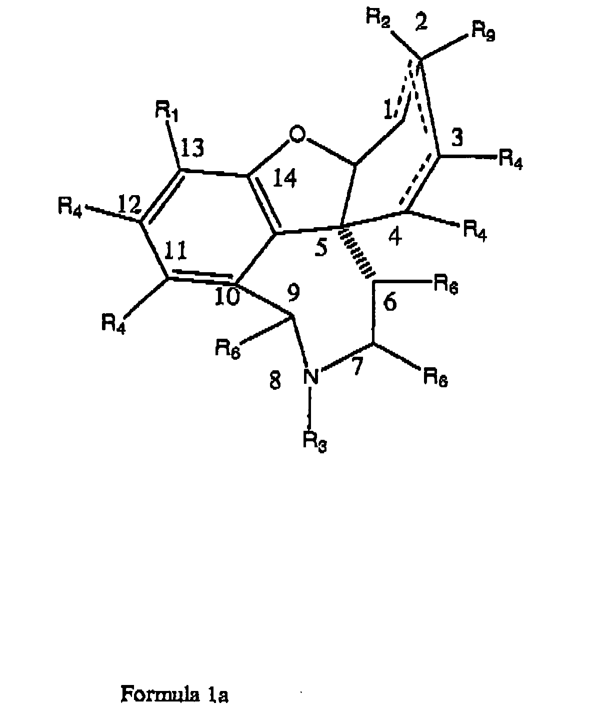 wherein the broken line represents an optionally present double bond in one or the two of the portions shown,
at least one of R₁ and R₂ is an aliphatic or aryl carbamate group wherein the aliphatic or aryl moiety maybe R₅ substituted or unsubstituted,
and the other of R₁ and R₂ is selected independently from the group consisting of hydrogen, hydroxyl, cyano, sulfhydryl, alkoxy of 1-6 carbon atoms, aryloxy, R₅-substituted aryloxy, aralkoxy, R₅-substituted aralkoxy, aryloxymethyl, R₅-substituted aryloxymethyl, alkanoyloxy, hydroxy-substituted alkanoyloxy, benzoyloxy, R₅-substituted benzoyloxy, aryloxy carbonyl and R₅-substituted aryloxy carbonyl, R₁ may also be alkyl of up to 14 carbon atoms, or hydroxy methyl, R₂ may also be carboxymethyl provided that at least one of R₁ and R₂ is hydroxy,
R₃ is hydrogen, straight or branched chain alkyl, of 1-6 carbon atoms, cycloalkylmethyl, phenyl, R₅ substituted phenyl, alkylphenyl,R₅-substituted alkylphenyl, heterocyclyl selected from α- or β-furyl, α or β-thionyl or thienyl, pyridyl, pyrazinyl and pyrimidyl, alkyl heterocyclyl or R'-substituted heterocyclyl, where heterocyclyl is as hereinabove defined and R' is alkyl or alkoxy
each R₄ is independently selected from hydrogen, hydroxyl, sulfhydryl, alkyl, aryl, aralkyl, alkoxy, mercaptoalkyl, aryloxy, alkaryloxy, nitro, amino, N-alkylamino. N-arylamino, N-alkarylamino, fluoro, chloro, bromo, iodo,
R₅ is selected from the same groups as R₄,
R₆ is hydrogen,
R₉ is hydrogen or alkyl of 1 to 6 carbon atoms, or is non-existant when the carbon in the 2 position is attached to a double bond
or a pharmaceutically-acceptable acid addition salt thereof

When there is no unsaturation in the 1,2 carbon bond, r2 is preferably oriented axially to the cyclohexane ring.

Preferably the aryl groups are phenyl groups, the aryloxy groups are phenoxy groups, the aralkyl groups are benzyl groups and the aralkoxy groups are benzyloxy groups.

Preferred compounds include those wherein R₁ and R₂ arc each selected from H, OR, SH, 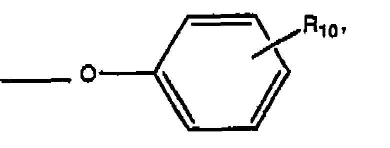 -OC(O)R, 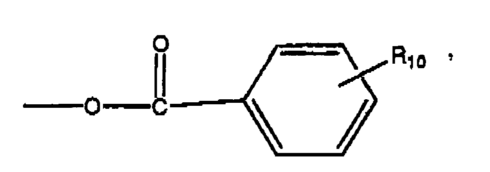 -OC(O)OR, 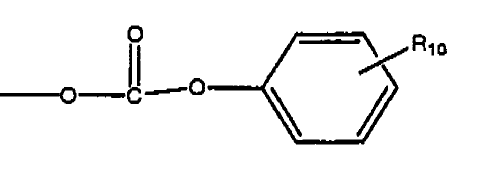 wherein R is alkyl 1-6 carbon atoms or phenyl or R₅- substituted phenyl or benzyl or
R₅-substituted benzyl, wherein R₁₀ is hydrogen, alkyl or alkoxy, R₃ is -H, or branched on linear alkyl or 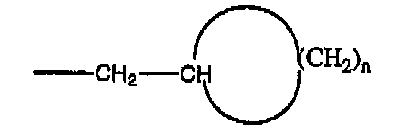 wherein n is 3, 4 or 5 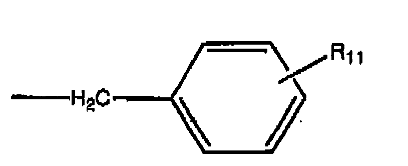 whereinR₁₁ is as defined for R₁₀ above, 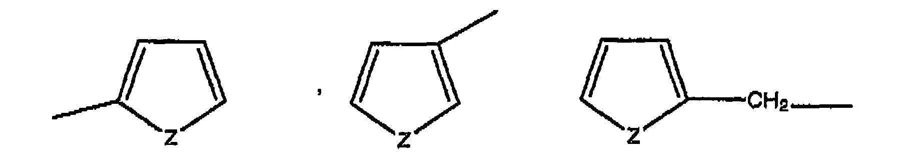 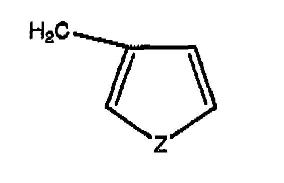 where Z is O, S, or NH.

When used herein the term "lower alkyl" means alkyl of 1-6 carbon atoms, preferably 1 - 4 carbon atoms, most preferabbly methyl or ethyl.

One class of compounds according to the present invention are those of the formula: 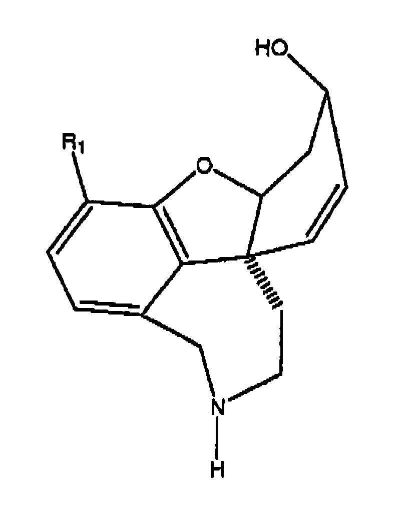 wherein R₁ is is as defined above for formula I, preferably hydroxy, lower alkoxy or aryloxy, an alkyl or aryl carbamyl group. Alternatively, R1 may be an alkyl or aryl carbarnate group as specified with respect to formula Ia.

For example, such compounds include O-demethyl, N-demethylgalanthamine; O-ethyl, O-demethyl, N-demethylgalanthamine; O-phenyl, O-demethyl, N-demethylgalanthamine; and O-benzyl, O-demethylgalanthamine. Useful carbamates of a similar structure may include phenylcarbamyl O-demethyl, N-demethylgalanthamine; mono [alpha]-naphthylcarbamyl O-demethyl N-demethyl galanthamine and dimethylcarbamyl O-demethyl, N-demethylgalanthamine,

A second class are those of the formula; 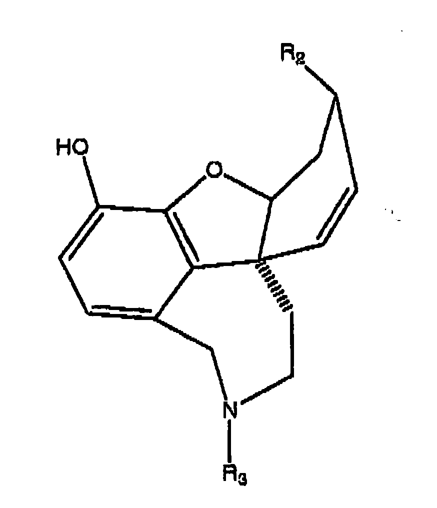 wherein R₂ is hydroxy, C₁₋₆ alkoxy, aryloxy, and R₃ is hydrogen or alkyl of 1-6 carbon atoms such as methyl ethyl, methyl cyclopropyl, benzyl or R₅-substituted benzyl. Other useful compounds are those wherein R₂ is an alkyl or aryl carbamate.

Such compounds include O-demethyl galanthamine; O-demethyl galanthamine; O-methyl ether; O-demethyl galanthamine; O-ethyl ether; O-demethyl galanthamine, O-benzyl ether; O-demethyl galanthamine, phenyl and O-demethyl N-demethyl galanthamine, α-naphthyl carbamates; O-demethyl galanthamine dimethyl carbamate and 0-dimethyl galanthamine diethyl carbamate wherein the carbamyl group is bonded to the oxygen of the cyclohexene ring, and the corresponding N-demethyl and N-demethyl N-ethyl and N-demethyl N-cyclopropyl methyl and N-demethyl N-benzyl compounds.

A third class of compounds comprises those of the formula: 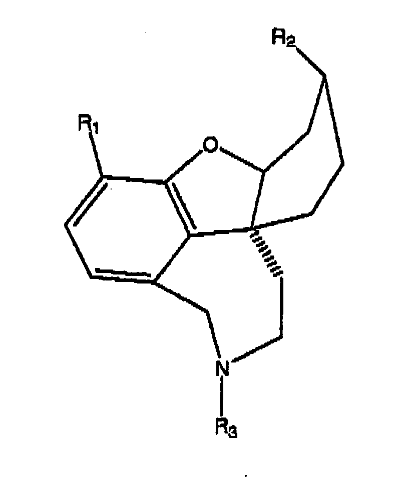 wherein R₁, R₂ and R₃ are as defined above for formula 1 or formula 1a, R₁ typically being hydroxy, C₁-C₆ alkoxy, benzyloxy or R₅-substituted benzyloxy, or alkyl or aryl carbamyl, R₂ is hydroxy, lower alkoxy, aryloxy, benzyloxy or an alkyl or aryl carbamyl group but is preferably hydroxy and R₃ is typically hydrogen, methyl, ethyl, cyclopropylmethyl, or benzyl.

Such compounds include, for example, O-dermthyl lycoramine; N-demethyl, O-demethyl lycoramine; N-demethyl N-ethyl lycoramine; N-demethyl-N-cyclopropylmethyl lycoramine; N-demethyl N-benzyl lycoramine; O-demethyl lycoramine ethyl ether; dooxy O-demethyl lycoramine; O-deoxy demethyl lycoramine, benzyl ether and dimethyl and phenyl carbamyl analogs of such compounds.

A further class comprising compounds of the formula: 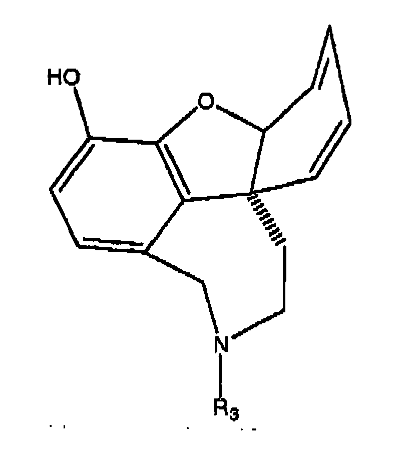 wherein R₃ is selected from hydrogen, lower alkyl, cycloalkyl methyl or benzyl.

The galanthamine carbamates discussed above are believed to be novel.

Useful compounds may include the following:

### GALANTHAMINE ANALOGS

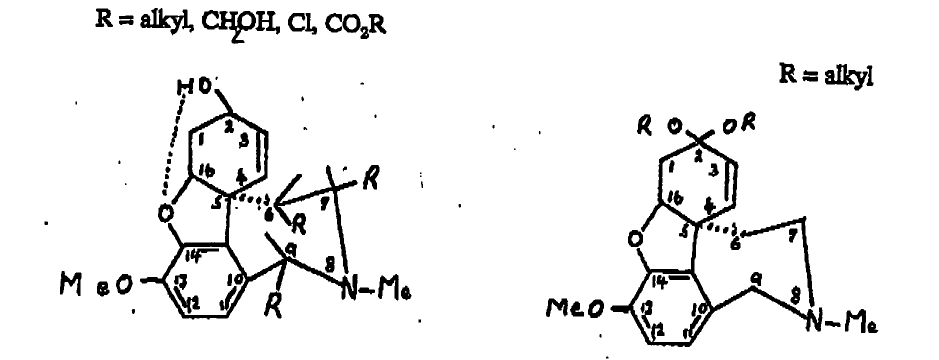

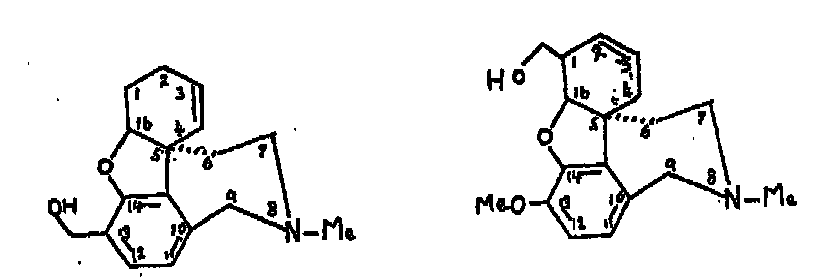

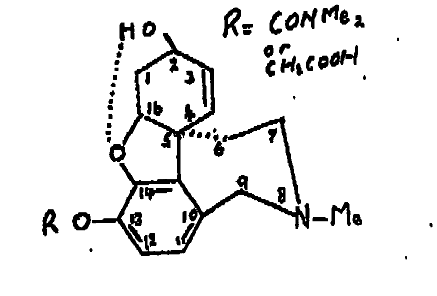

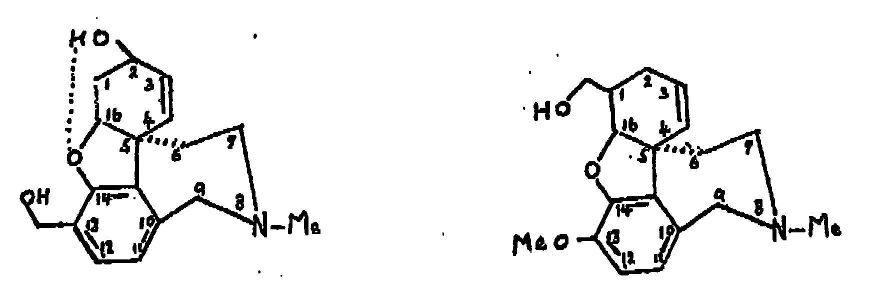

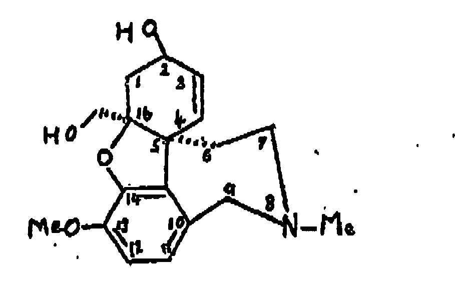

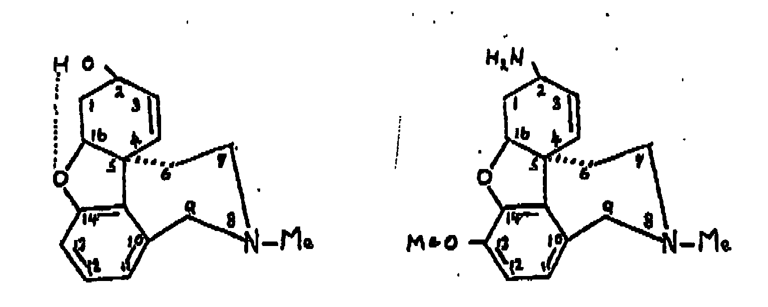

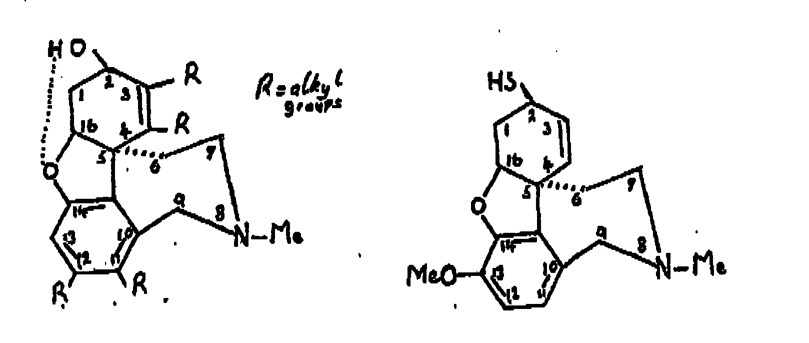

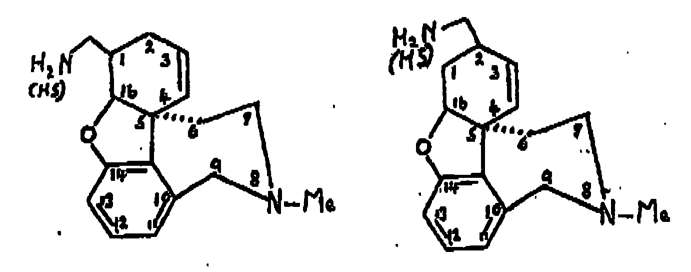

Many compounds of the present invention may be obtained by effecting suitable conversions of galanthamine. Galanthamine has the structure:

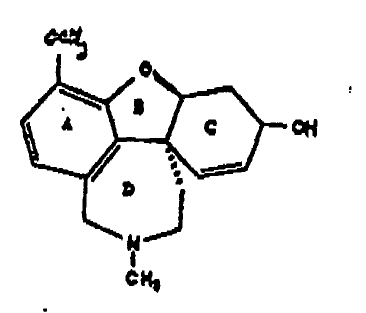

Compounds wherein R₁ is other than methoxy can be obtained from galanthamine by demethylation to the corresponding phenol and, if desired, effecting subsequent conversion thereon. Demethylation may be effected with iodotrimethylsilane in accordance with the method described by M.E. Jung and H.A. Lyster in J. Org. Chem. 42 3761 (1977). Reaction with iodotrimethylsilane may be effected in any convenient solvent, for example, chloroform at moderate temperatures (typically 25-40°C) for several hours (e.g. 12-20 hours) to cleave the methyl group.

The phenol obtained by this reaction may be employed itself or used as an intermediate for the production of other active compounds. If it is desired to avoid effecting the same reactions at the allylic hydroxy group in the C ring as are to take place at the phenol group that is produced by demethylation of the A ring when carrying out further interconversions, it is desirable to protect the allylic hydroxyl group. Suitable protection can be effected by converting the allylic hydroxyl group to its tetrahydropyranyl ether or 4-methoxytetra-hydropyranyl ether. Such ethers can be formed by reaction with dihydropyran or 4-methoxytetrahydropyran in a solvent such as dichloromethane at

room temperature in the presence of a strong organic acid such as p-toluenesulfonic acid. When desired, the protection groups can be removed, the tatrahyropyranyl group, for example, by treatment with methanol and Dowex-50 WXS and the 4-methoxytetrahydropyranyl group by reaction with very dilute (e.g., 0.01N) hydrochloric acid.

The phenolic group obtained by demethylation of the methoxy group of galanthamine may readily be converted into an alkali metal salt by reaction with sodium or potassium hydroxide or reaction with sodium hydride in tetrahydrofuran. The salt so obtained may be "alkylated" by reaction with an appropriate alkyl, aryl, alkaryl or R₅-substituted aryl or alkaryl or heterocyclyl or R₅-substituted heterocyclyl halide to produce a compound wherein R₁ is alkoxy (other than methoxy), aryloxy, alkaryloxy, R₅-substituted aryloxy, R₅-substituted alkoxy or heterocyclyl or R₅-substituted heterocyclyl. The reaction with the halide is typically run with excess halide in the absence of solvent or in a solvent such as dimethyl formamide or dimethylsulfoxide. For less reactive halides, the presence of a silver oxide catalyst may be desirable.

Reaction of the phenolic group with an isocyanate can be used to introduce a monoalkyl or monoaryl carbamyl group.

Dialkyl or diaryl carbamates may be obtained [by] from the mono alkyl or aryl carbamates by reaction with sodium hydride and an alkyl or aryl iodide. For example, the dimethyl carbamate can be obtained by reaction of the hydroxy group with methyl isocyanate followed by reaction with sodium hydride and methyl iodide.

Conversion of the phenolic group to an amino group, which can subsequently be alkylated by conventional means to produce a secondary amine if desired, can be effected by the Bucherer reaction using sodium bisulfite and ammonia.

The phenol group may be esterified, for example, with an acid anhydride or acid halide to produce an alkanoyloxy or aralkanoyloxy, R₁ group.

Production of compounds wherein R₁ is sulfhydryl can be effected by first converting the phenolic hydroxyl group obtained by demethylation of galanthamine to a thiol group. This can be effected, for example, by the Newman-Kwart rearrangement described, for example, in S. Patai ed "The Chemistry of the Thiol Group" Part 1 John Wiley & Sons, New York 1974 pages 201-206. This rearrangement is effected in three steps:
(1) conversion of the hydroxyl group to the O-aryl dialkylthiocarbamate by treatment with dialkylthiocarbonyl chloride;
(2) pyrolysis of the O-aryl dialkylthiocarbamate to the S-aryl dialkylthiocarbamate; and
(3) hydrolysis of this product to the aryl mercaptan.

The first stage reaction with dimethylthiocarbamyl chloride may be effected by dissolving the phenol obtained by demethylation of galanthamine in aqueous potassium hydroxide at 10°C or below and then reacting this with dimethylthiocarbamyl chloride in tetrahydrofuran at temperatures not exceeding 12°C. The solution is made alkaline and the O-aryl dimethylthiocarbamate is separated. This compound is pyrolyzed at 270-275°C for about 45 minutes in a salt bath, and treated with potassium hydroxide in ethylene glycol. The reaction is cooled, the product extracted and worked up.

The production of the thiol group also provides a convenient route for the production of compounds wherein R₁ is hydrogen. The thiol may be desulfurized, for example, by refluxing with Raney nickel in absolute alcohol or dioxane, for example, as described by R.L. Augustine "Catalytic Hydrogenation" Marcel Dekker Inc., New York 1965 pp 131-133.

Production of a galanthamine analogue where R₂ is an alkanoyloxy or benzoyl group can be obtained by a simple esterification reaction. Compounds wherein R₂ is alkyloxy, aryloxy, aralkyloxy or alkaryloxy may be formed by forming a salt of the alcohol by reaction with sodium and thereafter reacting the salt with alkyl or other halide as described above for alkylating a phenol salt to produce R₁ as other than methyl.

Similarly, reaction of the hydroxy group with an aryl or alkyl isocyanate will produce compounds wherein R₂ is a monoaryl or monoalkyl carbamate

In other transformation of the R₂ group, the first step in modification will normally be conversion of the allylic alcohol into an allylic bromide. This may be effected by reaction with a slight excess of carbon tetrachloride and triphenylphosphine in a solvent such an methylene chloride at reduced temperature, for example, around 0°C. The bromide may then be reacted with magnesium in a Grignard reaction and the Grignard reagent obtained reacted with water to produce a compound wherein R₂ is hydrogen. Alternatively, reaction of the allylic bromide with lithium aluminum hydride may achieve the same product.

The allyl bromide may also be used as an intermediate for the introduction of other groups into the C ring. For example, the bromide will react with nucleophiles such as sodium or potassium hydrosulfide to replace the bromo group by a hydrosulfuryl group or with sodium cyanide to introduce a cyano group. The hydrosulfuryl group may be converted into a salt and alkylated in the same ways as can a hydrosulfuryl group in the A ring.

The allylic hydroxyl group may also be converted into a keto group. This can be accomplished, for example, by reaction with Jones reagent (H₂CrO₄, H₂SO₄ water and acetone). Proceeding via the keto group may also be an alternative route to production of compounds wherein R₂ is hydrogen. For example, the keto compound may be reacted with ethane dithiol and boron trifluoride etherate and the 1,3-dithiolane produced then desulfurized by reaction with Raney nickel.

The ketone intermediate may also be used as a source for compounds wherein R₂ is amino, such compounds being obtained by reductive amination of the keto group with ammonia and hydrogen in the presence of a nickel catalyst.

Further analogues that can be obtained by use of the ketone as an intermediate are those wherein R₉ is other than hydrogen. These can be obtained by reaction of the ketone with a Grignard reagent.

The ketone may also be used as an intermediate for the production of compounds wherein R₇ is hydroxy methyl by first effecting an α-bromination of tha ketone and then converting this to hydroxy methyl.

When R₂ and R₉ jointly form a semi carbazone this can be formed from the ketone by reaction with semi carbazide.

In order to produce compounds wherein R₃ is other than methyl, galanthamine is first demethylated to produce a compound wherein R₃ is hydrogen. Demethylation may be effected by reaction with a chloroformate such as methyl chloroformate or phenyl chloroformate to produce a carbamate which may then be cleaved with hydrazine, or by reaction with β,β,β-trichloromethyl chloroformate followed by reaction with zinc and acetic acid. The resulting amine may then be alkylated with other alkyl groups, branched or unbranched, alkylphenyl group or alkylheterocyclic groups. This reaction may be carried out by converting -NH to the corresponding sodium or potassium salt (NaK, KH) and treating the salt with the corresponding halide, preferably iodides, but bromides and chlorides might be used. All the halides used are commercially available. The reaction conditions may be modified for less reactive halides such as chlorides or aryl or heterocyclic halides which may also be less reactive. For instance, a special method is needed for N-phenylation as described by D.H.R. Barton, J.P. Finet and J. Khamsi, Tetrahedron Letters 28, 887 (1987).

It is also possible to change the degree of saturation of the "core" structure of galanthamine. For example, if one forms an allylic bromide in the C-ring as described above, this bromide may be subject to catalytic hydrogenation in the presence of palladium, for example, palladium on carbon in ethanol at room temperature and atmospheric pressure to remove not only the bromine atom, but also to saturate the unsaturated bond in the C ring.

The C-ring may be oxidized to produce a further unsaturated bond by oxidation, for example, by heating with nickel, platinum or palladium at 300-350°C or possibly under milder conditions if acceptors such as maleic acid, cyclohexane or benzene are present.

Conversion of R₈ to hydroxy methyl may be effected by photolyzing in the presence of cyanogen chloride to introduce a cyano group. This may be reduced using a Ranez nickel catalyst to produce an aldehyde group that can itself be reduced to hydroxy methyl.

Similar conversions can be effected using lycoramine as starting material to produce compounds wherein the C ring is saturated.

In addition to modifying galanthamine, compounds according to the present invention may also be produced by cyclizing an amide of the formula: 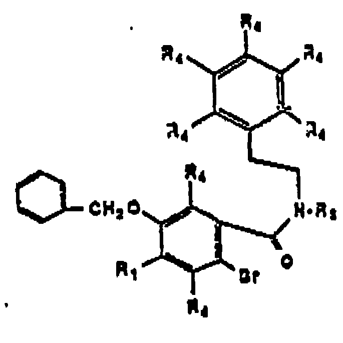 R₁,-R₃ and R₄ groups may be selected as desired in the starting material employed. Cyclization is effected by electrochemical oxidation of the type described in U.S. Patent 4,290,862 for preparing narwedine-type enones. The linear precursor in an appropriate solvent with a conductive salt and 2% HBF₄ or KClO₄ or K₂CO₃ is added to the anode compartment of an electrolytic cell. The cathode compartment and the electrolytic bridge of the reference electrode contains the same anodic solvent and the same percentage of conductive salt. The working electrode is platinum. Oxidation is carried out at 1.3 volts at low temperature (below 0°C). This procedures after workup of the product affords cyclization products.

The compounds of the present invention may be used for the treatment of Alzheimer's disease either in free base form or as the acid addition salts.

The compounds can be administered in any convenient chemical or physical form. For example, they may be administered as pharmaceutically acceptable salts, as long as these do not quaternise the D-ring nitrogen atom. Useful salts include the hydrobromide and hydrochloride.

The compounds or the pharmaceutically-acceptance acid addition salts may be administered to a patient suffering from Alzheimer's disease orally or by subcutaneous or intravenous or injection. Sustained release delivery mechanisms are particularly useful for administration of the compounds of the present invention, for example, intracerebroventricularly by means of an implanted reservoir by use of sustained release capsules or by means of a trans dermal patch. It may be necessary to begin at lower doses than are ultimately effective.

Certain of the compounds may be only sparingly soluble in water at room temperature and so injectable compositions are normally in the form of an aqueous suspension. If necessary, pharmaceutically-acceptable suspension aids may be employed. Typically, such a suspension will be employed at a concentration of 1-50 mg/ml more commonly 5-40 mg/ml, for example, 5-30 mg/ml or 10-40 mg/ml, typically 20-30 mg/ml of the compound of the present intention. Typical dosage rates when administering compounds of the invention will depend upon the exact nature and condition of the patient. For example, typical dosage rates for administration by injection are in the range 5-1,000 mg per day depending upon the patient. In some cases, even lower dosages such as 0.5 or 1 mg per day may be helpful. For example, divided doses in the range 0.5-5 mg/kg body weight per day may prove useful. Typically, one might administer a dosage of 50-300 mg per day to a patient of a body weight of 40-100 kg, although in appropriate cases such dosages may prove useful for patients having a body weight outside this range. In other cases, dosages as low as 10 mg and as high as 500 mg may be appropriate for persons in this body weight range.

The compounds of the invention may also be administered orally, for example, as an aqueous suspension or a solution in aqueous ethanol or as a solid such as a tablet or capsule. Suspensions or solutions for oral administration are typically of about the same concentration as those used for injections. However, it may be desirable when administering the drug orally to use a higher dosage rate than when administering it by injection. For example, dosages up to 2000 mg per day may be used, such as dosages in the range 100-600 mg per day. In preparing such tablets or capsules, standard tablet or capsule-making techniques may be employed. The dosage rate of the compound of the invention or its pharmaceutically-acceptable salt will normally be in the same range as for oral administration of a liquid. If desired, a pharmaceutically-acceptable carrier such as starch or lactose may be used in preparing tablets. Capsules may be prepared using soft gelatine as the encapsulating agent. If desired, such capsules may be in the form of sustained release capsules wherein the main capsule contains microcapsules of active compound which release the contents over a period of several hours thereby maintaining a constant level of active compound in the patient's blood stream.

The following test provides a good animal model for Alzheimer's disease in humans: A selective lesion is placed in a subcortical nucleus (nucleus basalis of Meynert) with a resultant cortical cholinergic deficiency, similar in magnitude to that seen in early to moderate stage Alzheimer's disease. Numerous behavioral deficits, including the inability to learn and retain new information, characterizes this lesion. Drugs that can normalize these abnormalities would have a reasonable expectation of efficacy in Alzheimer's disease. Haroutunian, V, Kanof P, Davis, KL: Pharmacological alleviations of cholinergic-lesion-induced memory defects in rats. Life Sciences 37:945-952, 1985.

The following specific formulations may find use in treatment of Alzheimer's disease:

Tablets or capsules containing 5, 10 and 25 mg hydrobromide of a compound according to the invention to be taken four times a day, or a sustained-release-preparation delivering an equivalent daily dose.

Parenteral solution containing 5 mg/ml.

Liquid formulation for oral administration available in 5mg/5ml and 25mg/5ml concentration.

There have been reports that galanthamine can cause cardiac arrythmias. If such problems occur with the compounds of the present invention, it may be desirable to administer compounds of the invention in conjunction with another drug such as propanthelinbromide to control such arrythmias. As with other drugs that act on the central nervous system, minor aide effects, such as nausea, may be noted. In this case, the compounds of the present invention will be administered in conjunction with agent for control of such side effects.

A substantial proportion of patients suffering from Alzheimer's disease exhibit not only reduced levels of acetyl choline but also of norepinephrine in the brain. In such cases, the compound of the present invention may advantageously be employed in conjunction with compounds such as clonidine desipramine, monoamine oxidase inhibitors, methamphetamine and methyl phenidate that stimulate the noradrenergic receptors in the brain.

### PROCEDURES:

### 1. Conversion of oxygen to nitrogen in furanoid ring.

This transformation has been accomplished by several authors. Passage of furan and its homologs, or reduced furans with ammonia over alumina, at 400-450°C, affords the corresponding pyrroles. With primary, amines the N-substituted pyrrole is obtained. A typical procedure is as follows. The furan derivative is dissolved in liquid ammonia and passed over an alumina catalyst (200 c.c. 4-6 mesh) which has been preheated to 400°C.¹ Alternatively, a mixture of a furan derivative and ammonia in aqueous-alcoholic medium may be heated at temperature around 110°-150°C. The reaction proceeds more readily under pressure and therefore can be carried out in an autoclave.²
1. C.L. Wilson, J. Chem. Soc. 63. 1945.
2. R.C. Fuson, C.L. Fleming, R. Johnson, J. Am. Chem. Soc. 60, 1994 (1938).

### 2. Introduction of -CH₂OH at position 16.

Position 16 is next to the furan ring. This position is equivalent to the α-position of an ether and therefore prone to radical attack by reagents such as oxygen, peroxides, and photochemical reactions.

Irradiation of cyclic ethers in the presence of cyanogen chloride is known to produce α-cyano ethers in good yields.¹ Therefore, a similar reaction on galanthamine should introduce a cyano group in this position. The cyano group is known to be converted to an aldehyde with Raney Nickel and formic acid.² The aldehyde may in turn be reduced with any reducing agents to the hydroxymethyl group.³
1. E. Muller, H. Huber, Chem. Ber. 96, 2319 (1963).
2. T. van Es, B. Stakun, J. Chem. Soc. 5775 (1965).
3. S.R. Sandler, W. Karo, Organic Functional Group Preparations", Vol 12, 1968. Academic Press, pp. 89-90.

A typical procedure is as follows. The photolysis is carried out using a mercury lamp S-81 in a water cooled, unfiltered quartz immersion well. A solution of equimolar amounts of galanthamine and cyanogen chloride, in a spectral grade solvent, is irradiated with stirring, in a nitrogen atmosphere, and in a quartz cell. The reaction is irradiated for 2h in the presence of sodium bicarbonate. The sodium bicarbonate is essential to absorb the hydrogen chloride. Lower yields are obtained in the absence of the reagent.

The final mixture is filtered, concentrated, washed with appropriate reagents and dried. Removal of solvent affords the product.

The cyano derivative obtained is dissolved in 75% (v/v) of aqueous formic acid and treated with a 50:50 Ni-Al alloy (Raney type). The mixture is heated for 3h at 95°C, diluted with absolute ethanol-ethyl acetate and filtered through Celite. The filtrate is concentrated, washed with appropriated reagents and dried. Removal of solvent affords the product.

The aldehyde derivative is dissolved in isopropanol and added to a solution of sodium hydroxide and sodium borohydride, dropwise, at such rate that the reaction refluxes gently. After standing overnight, the reaction is worked up as usual.

### 3. Conversion of ·OMe at position 13 to other substituents.

The reaction will require demethylation of the methoxy group. Although demethylation of aromatic alkoxy group is considered a standard reaction, this operation may be tricky in polysubstituted systems. A successful approach is the reaction of boron tribromide at 0°C.¹ The phenol obtained may be converted to a bromide by standard procedure.² Generation of a lithium anion at this position³ allows a variety of reactions such as treatment with alkyl halides to give alkyl groups, carbon dioxide to afford acids or aldehydes and ketones to give alcohols.
1. S-Y Han, J.T. Gordon, K. Bhat, M.B. Dratman, M.M. Joullie', Int. J. Peptide Protein Res. 30, 1987, 652.
2. C.E. Kaslow and M.M. Marsh, J. Org. Chem. 12, 456 (1947).
3. J. Chiarello and M.M. Joullie', Tetrahedron, 44, 41 (1988).

A typical procedure follows. A 1M solution of boron tribromide is added dropwise to a solution of galanthamine in dry methylene chloride at 0°C, under nitrogen. After stirring at 0°C for 3h, the excess reagent and boron complexes are hydrolyzed by water. The product is obtained by extraction of the aqueous layer with ether. Removal of the solvent affords the compound.

The phenol derivative is mixed thoroughly with phosphorus pentabromide and heated at 70-80°C, then at 120°C. Hydrolysis with ice and water affords the crude bromo compound which may be recrystallized from an appropriate solvent.

A solution of n-butyl lithium was added dropwise, at -78°C, under argon, to the bromo derivative dissolved in tetrahydrofuran and hexamethyl phosphoramide to generate the intermediate anion.

To this anion, one may add any alkyl halide to form an derivative. Alternatively, the solution could be poured over dry ice to afford the corresponding carboxylic acid, or to an aldehyde or ketone to form the corresponding alcohol derivative. Treatment of this anion with ethyl chloracetate would give the corresponding ethyl carboxymethyl derivative. The above methodology is general to introduce various groups in place of the methoxy group in the aromatic ring (position 13).

### 4. Conversions in the D ring

Removal of the methyl group in the D ring of galanthamine can be accomplished by the classical von Braun reaction. 1 The reaction may be manipulated to give either demethylation to a secondary amine or to open the ring. Either of these procedures will give rise to new analogs. Ring opening would afford two chains, one at position 10, possibly a bromoethyl groups and one at position 5, a N-methyl propylamine. These could be used to study the effect of disrupting ring D.

However, demethylation would serve to introduce functionality in ring D *via* the formation of an imine. A typical procedure for introduction of substituents in ring D follows. Treatment of N-demethylated galanthamine with tert-butyl hypochlorite at 0°C will afford the N-chloro derivative. This sensitive compound is not isolated but immediately dehalogenated with either sodium methoxide or diazabicycloundecene to afford the highly unstable pyrroline. This intermediate can be treated with nucleophiles³ or with benzoic and, *tert*-butylisonitrile as described in reference 2.
1. H.A. Hagerman, Org. Reactions, VII, Chap 4 (1953).
2. R.F. Nutt, M.M. Joullie', J. Am. Chem. Soc., 104, 5852 (1982).
3. J. Hausler, U. Schmidt, Liebigs Ann, Chem. 1881 (1979).

The production of compounds of the present invention is illustrated by the following Examples:

### Reference EXAMPLE 1

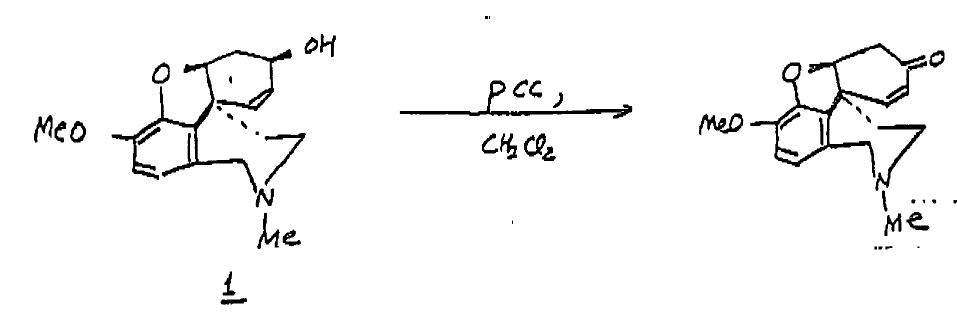

To a solution of Galanthamine (1, 0.7g, 0.2439 mmoles) in dry methylene chloride (4 ml), pyridinium chlorochromate (0.1577g, 0.7317 mmoles) was added and stirred at room temperature for 8 hours. The reaction mixture was diluted with methanol and filtered. Removal of solvent followed by purification on a silica gel column using acetone, methanol:acetone (10:90) as eluants afforded the desired product. (0.06g, 86% yield). mp. 184-186°C

### Reference EXAMPLE 2

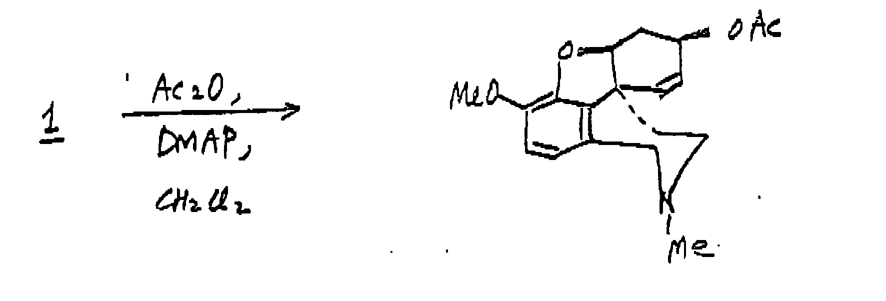

To a solution of 1 (0.07g, 0.2439 mmoles) in methylene chloride, acetic anhydride (0.03 ml, 0.317 mmoles) and dimethylaminopyridine (0.0536g, 0.439 mmoles) were added at 0°C in an ice bath. The reaction mixture was stirred at 0°C for 10 minutes and room temperature for 60 minutes. Solvents were removed on a rotary evaporator and the residue was diluted with ethyl acetate, washed with water, 10% Na₂CO₃, brine and dried (Na₂SO₄) concentration of the solvent and purification by silica gel chromatography using Acetone, MeOH:Acetone (1:10) as eluants afforded 0.0773g of product (96% yield).
mp. 126-128°C

### EXAMPLE 1

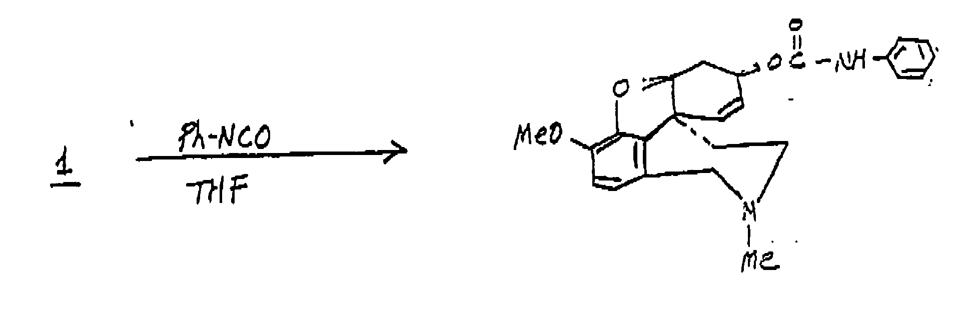

Phenylisocyante (0.033 ml, 0.2926 mmoles) was added to a stirred solution of 1 (0.07 g, 0.2439 mmoles) in the THF (5 ml) at room temperature, and than stirred for 30 hours at the same condition. The reaction mixture was concantratad, purified by silica gel column chromatography using Acetone, MeOH:Acetone (10:90) to give 0.0982g (99% yield) of product. mp 79-81°C

### EXAMPLE 2

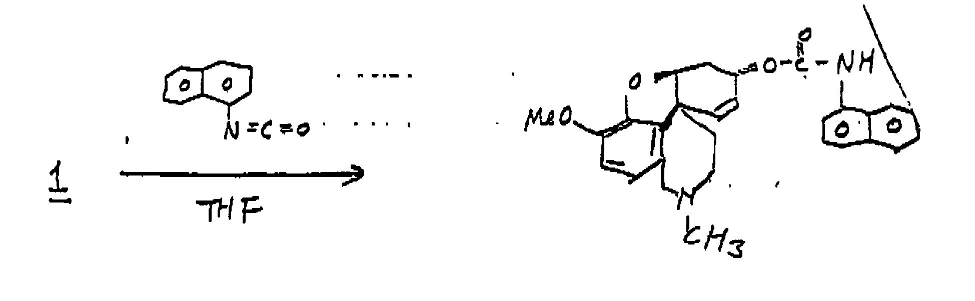

1-Naphthyl isocyante (0.042 ml, 0.2926 mmoles) was added to a stirred solution of 1 (0.07g, 0.2439 mmoles) in THE (5 ml) at room temperature. The reaction mixture was stirred for 24 hours at room temperature, and then concentrated. The crude reaction mixture was purified by silica gel column chromatography using acetone, methanol:acetone (10:90) as eluants to afford 0.11g of product (99%).
mp. 198-200°C

### Reference EXAMPLE 3

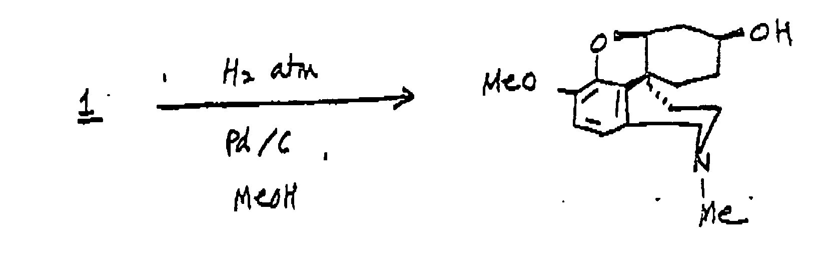

To a solution of 1 (0.1g, 0.3484 mmoles) in methanol, 20% palladium on carbon (0.02g) was added and hydrogen atmosphere was applied. The reaction mixture was stirred for 10 hours at room temperature. The catalyst was filtered off through celite, washed thoroughly with methanol. The solution was concentrated, and the crude material was purified on a silica gel column using acetone:methanol (90:10, 80:20) to afford 0.0938g (93% yield) of the product.
mp 110-112°C

Anticholinesterase activity of the compounds of the above examples was assayed by an assay for inhibition of acetylcholinesterase following the procedure of G. Ellman, Biological Pharmacology, 1961, Vol. 7, pp 88-95. Acetylthiocholine serves as substrate since it acts like acetylcholine. Acetylcholinesterase cleaves it to thiocholine and acetate which react with dithiobisnitrobenzoate to form a yellow color which is measured photometrically.

The assay results are shown in the following table, which also includes an assay of the activity of galanthamine.

**TABLE**

| COMPOUND | PERCENT INHIBITION OF ACETYLCHOLINESTERASE |
|---|---|
| Galanthamine | 95% |
| Produce of Reference Example 1 | 10% |
| Product of Reference Example 2 | 37% |
| Product of Example 1 | 37% |
| Product of Example 2 | 60% |
| Product of Reference Example 3 | 30% |

## Claims

1. Use of a compound of the formula I 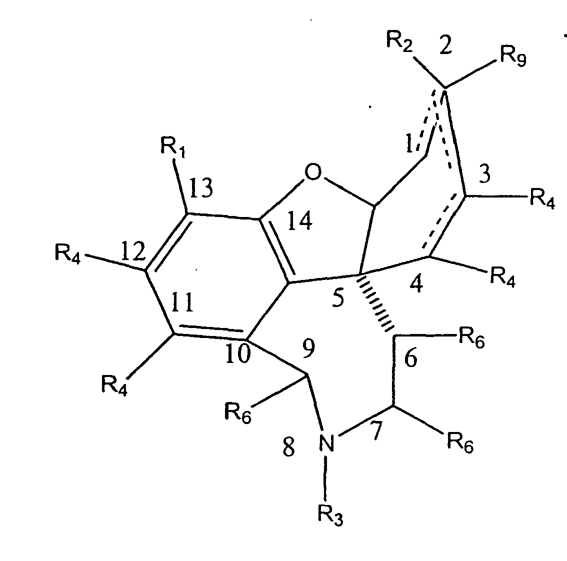 wherein the broken line represents an optionally present double bond in one or the two of the portions shown,
R₁ and R₂ are each selected independently from the group consisting of hydrogen, hydroxyl, , cyano, sulfhydryl, alkoxy of 1-6 carbon atoms, aryloxy, R₅substituted aryloxy, aralkoxy, R₅-substituted aralkoxy, aryloxymethyl R₅-substituted aryloxymethyl, alkanoyloxy, hydroxy-substituted alkanoyloxy, benzoyloxy, R₅-substituted benzoyloxy, aryloxy carbonyl and R₅-substituted aryloxy carbonyl",
R₃ is hydrogen, straight or branched chain alkyl of 1-6 carbon atoms, cycloalkylmethyl, alkylphenyl,R₅-substituted alkylphenyl, heterocyclyl selected from α- or β-furyl, α or β-thienyl or thenyl, pyridyl, pyrazinyl and pyrimidyl, alkyl heterocyclyl or R'-substituted heterocyclyl, where heterocyclyl is as hereinabove defined and R' is alkyl or alkoxy
each R₄ is independently selected from hydrogen, hydroxyl, sulfhydryl, alkyl, aryl, aralkyl, alkoxy, aryloxy, alkaryloxy, nitro, amino, N-alkylamino, N-arylamino, N-alkarylamino, fluoro, chloro, bromo, and iodo ,
R₅ is selected from the same groups as R₄,
R₆ is hydrogen,
R₉ is hydrogen, or is non-existant when the carbon in the 2 position is attached to a double bond with the proviso that with the proviso that R₃ is not methyl when R₁ is methoxy R₂ is hydroxy and all R₄ are hydrogen and with the proviso that on sheet 2a the compounds are not those wherein the hydroxy of galanthamine is replaced by methoxy, ethoxy, C₁-C₅ lower alkanoyloxy such as acetyloxy the methoxy group is replaced by hydrogen, methoxy, ethoxy or lower alkanoyloxy of from 1 to 5 carbon atoms such as acetyloxyon sheet 2a and the methyl group substituted on the nitrogen atom of galanthamie is replaced by other straight or branch chain C₁-C₅ lower alkyl groups such as ethyl, cyclopropylmethyl or cyclobutylmethyl, allyl, C₁-C₅ lower alkylphenyl or substituted C₁-C₅ loweralkylphenyl wherein the substituents are fluoro, chloro, bromo, C₁-C₅ lower alkoxy, hydroxy, nitro, amino, C₁-C₅ lower alkyl or acyl amino of from 1 to 5 carbon atoms, heteroaryl C₁-C₅ lower alkyl in which the heteroaryl group is thienyl, furyl, pyridyl, pyrrolyl, or pyrazinyl, or a cyano radical; or unsubstituted and halogen substituted benzoyl C₁-C₅ lower alkyl in which the substiuents are on the phenyl ring and compounds wherein hydrogen atoms in the "core" structure have been replaced by fluoro or chloro groups ;
or a pharmaceutically-acceptable acid addition salt thereof for the production of a medicament for the treatment of Alzheimer's disease.

2. A use according to claim 1 wherein the compound used is one wherein R₁ and R₂ are each selected from
H, OR, SH, 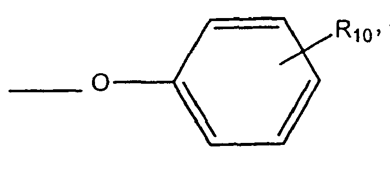 -OC(O)R, 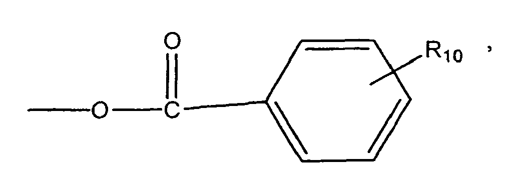 -OC(O)OR, 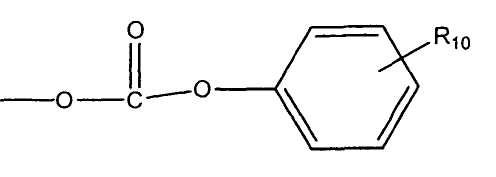 wherein R is alkyl 1-6 carbon atoms or phenyl or R₅- substituted phenyl or benzyl or R₅-substituted benzyl, wherein R₁₀ is hydrogen, alkyl or alkoxy, R₃ is -H, or branched on linear alkyl or 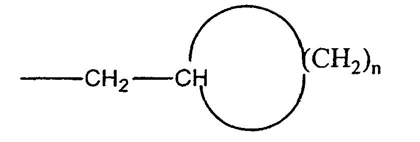 wherein n is 3, 4 or 5 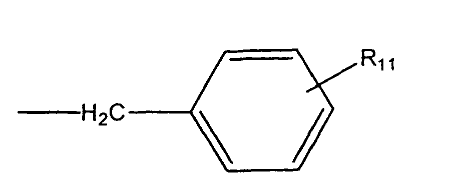 wherein R₁₁ is as defined for R₁₀ above 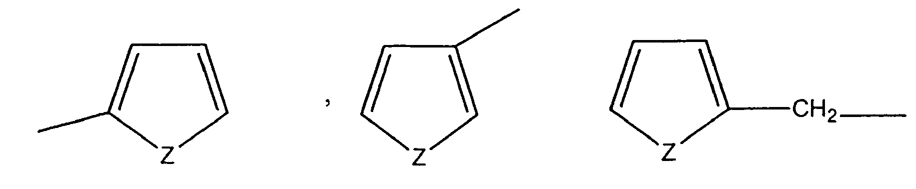 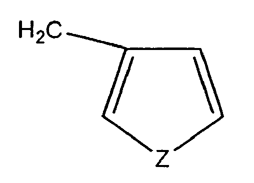 where Z is O, S, or NH.

3. A use according to claim 1 wherein said compound is of the formula: 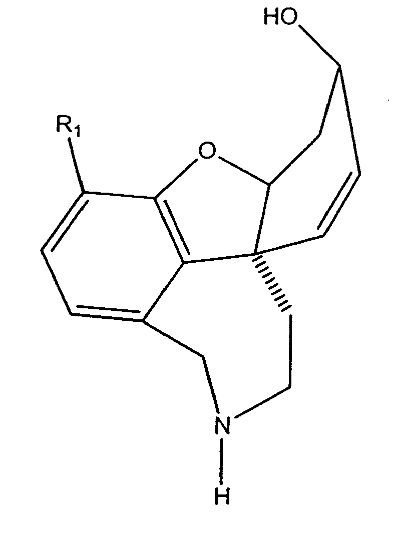 wherein R₁ is hydroxy, C₁-C₆ lower alkoxy, aryloxy, R₅ substituted aryloxy,

4. A use according to claim 3, wherein said compound is selected from O-demethyl, N-demethyl galanthamine; O-ethyl,O-demethyl, N-demethyl galanthamine; O-phenyl, O-demethyl, N-demethyl galanthamine; and O-benzyl,O-demethyl galanthamine.

5. A use according to claim 1 wherein said compound is of the formula: 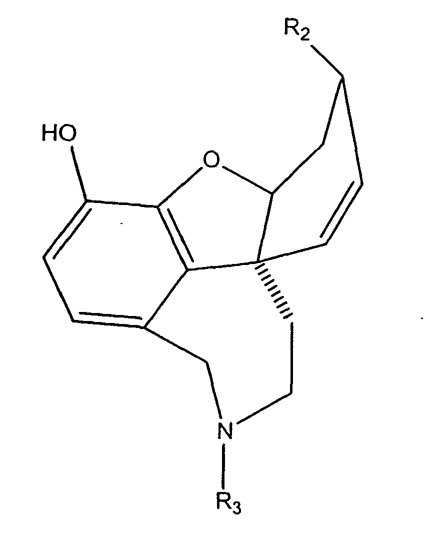 wherein R₂ is hydroxy, C₁-C₆ alkoxy, aryloxy, and R³ is hydrogen or alkyl of 1-6 carbon atons such as methyl or ethyl methyl cyclopropyl or benzyl or R⁵-substituted benzyl.

6. A use according to claim 5, wherein said compound is selected from O-demethyl galanthamine; O-demethyl galanthamine;O ethyl ether; - and the corresponding N-demethyl and Ndemethyl N-ethyl and N-demethyl N-cyclopropyl methyl and N demethyl N-benzyl compounds.

7. A use according to claim 1, wherein said compound is of the formula: 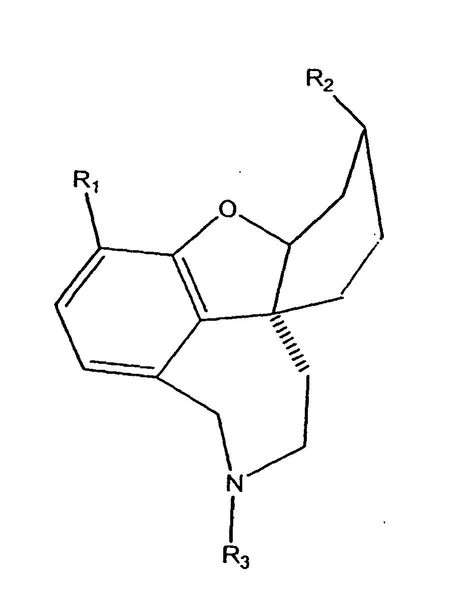 wherein R₁ R₂ and R₃ are as defined in claim 1.

8. A use according to claim 7, wherein the compound employed is one wherein R₁ is hydroxy, C₁-C₆ alkoxy, benzyloxy or R₅ substituted benzyloxy" R₂ is hydroxy, C₁-C₆ alkoxy, aryloxy, benzyloxy and R₃ is hydrogen, methyl ethyl, cyclopropyl methyl or benzyl.

9. A use according to claim 8, wherein said compound is selected from O-demethyl lycoramine; N-demethyl,O-demethyl lycoramine; N-demethyl N- ethyl lycoramine; N-demethyl Ncyclopropylmethyl lycoramine; N-demethyl N-benzyl lycoramine;O-demethyl lycoramine ethyl ether; deoxyO-demethyl lycoramine; O-deoxy demethyl lycoramine, and benzyl ether.

10. A use according to claim 1, wherein said compound is of the formula: 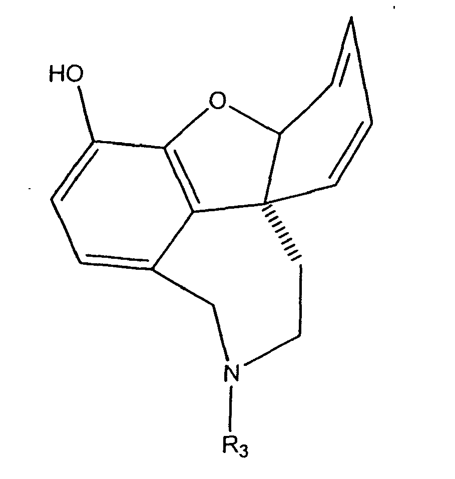 wherein R₃ is selected from hydrogen, lower alkyl of 1 to 6 carbon atoms, cycloalkyl methyl or benzyl.

11. A use according to any one of the preceding claims, wherein the administration is suitable for parenteral administration at a daily dosage of 0.5-1,000 mg of a compound of formula I as claimed in claim 1 or a pharmaceutically-acceptable acid addition salt thereof.

12. A use according to any one of the preceding claims, wherein said dosage rate is 50-300 mg per day.

13. A use according to any one of preceding claims 1 -10, wherein said medicament is suitable for oral administration and is in the range 10-2000 mg per day.

14. A use according to claim 13, wherein said dosage rate is of 100-600 mg per day.

15. A use according to any one of claims 1-10, wherein said medicament is suitable for parenteral administration at a dosage rate of 0.1 to 4 mg/kg body weight of a patient.

16. A use according to any one of claims 1-10, wherein said medicament is suitable for administration intracerebroventricularly via an implanted reservoir at a dosage rate of 0.01 to 5.0 mg/kg day.

17. A use according to any one of claims 1-10, wherein said medicament is in the form of a sustained release formulation.

18. Use of a compound of the formula I 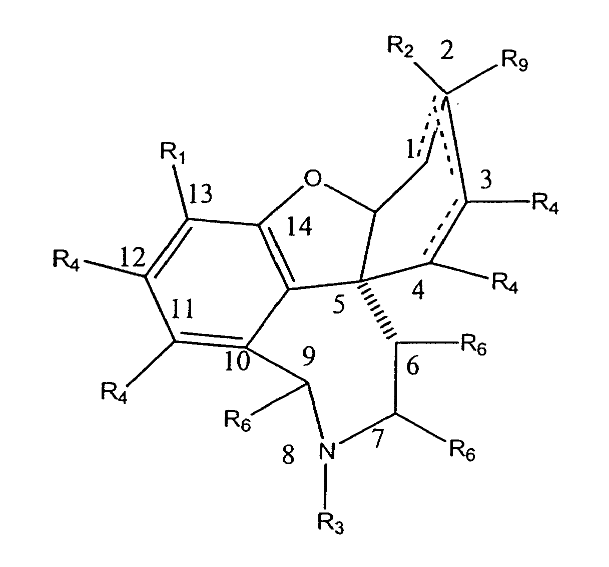 wherein the broken line represents an optionally present double bond in one or the two of the portions shown,
at least one of R₁ and R₂ is an aliphatic or aryl carbamate group wherein the aliphatic or aryl moiety may be R₅ substituted or unsubstituted,
and the other of R₁ and R₂ is selected independently from the group consisting of hydrogen, hydroxyl, cyano, sulfhydryl, alkoxy of 1-6 carbon atoms, aryloxy, R₅substituted aryloxy, aralkoxy,R₅-substituted aralkoxy, aryloxymethyl R₅-substituted aryloxymethyl, alkanoyloxy, hydroxy-substituted alkanoyloxy, benzoyloxy, R₅-substituted benzoyloxy, aryloxy carbonyl and R₅-substituted aryloxy carbonyl, R₁ may also be alkyl of up to 14 carbon atoms, or hydroxy methyl, R₂ may also be carboxymethyl provided that at least one of R₁ and R₂ is hydroxy,
R₃ is hydrogen, straight or branched chain alkyl, of 1-6 carbon atoms, cycloalkylmethyl, phenyl, R₅ substituted phenyl, alkylphenyl,R₅-substituted alkylphenyl, heterocyclyl selected from α- or β-furyl, α or β-thienyl or thienyl, pyridyl, pyrazinyl and pyrimidyl, alkyl heterocyclyl or R'- substituted heterocyclyl, where heterocyclyl is as hereinabove defined and R' is alkyl or alkoxy
each R₄ is independently selected from hydrogen, hydroxyl, sulfhydryl, alkyl, aryl, aralkyl, alkoxy, mercaptoalkyl, aryloxy, alkaryloxy, nitro, amino, N-alkylamino, N-arylamino, N-alkarylamino, fluoro, chloro, bromo, iodo,
R₅ is selected from the same groups as R₄,
R₆ is hydrogen,
R₉ is hydrogen or alkyl of 1 to 6 carbon atoms,or is non-existant when the carbon in the 2 position is attached to a double bond
or a pharmaceutically-acceptable acid addition salt thereof for the production of a medicament for the treatment of Alzheimer's disease.

19. A use as claimed in claim 18 wherein the compound used is one wherein R₁ and R₂ are each selected from
H, OR, SH, 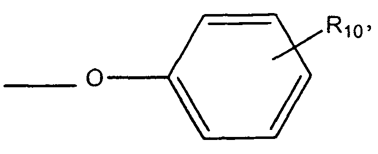 -OC(O)R, 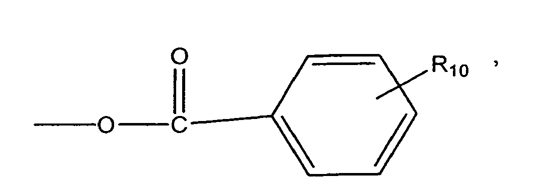 -OC(O)OR, 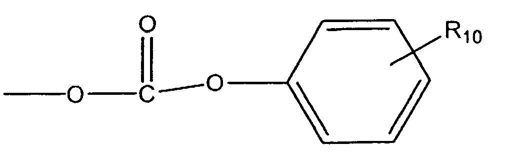 wherein R is alkyl 1-6 carbon atoms or phenyl or R₅- substituted phenyl or benzyl or R₅-substituted benzyl, wherein R₁₀ is hydrogen, alkyl or alkoxy, R₃ is -H, or branched on linear alkyl or 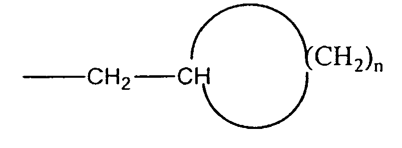 wherein n is 3, 4 or 5

20. A compound which is selected from O-demethyl galanthamine-phenyl carbamate and O-demethyl N-demethyl galanthamine, phenyl and -naphthyl carbamates; O-demethyl galanthamine dimethyl carbamate and O-dimethyl galanthamine diethyl carbamate wherein the carbamyl group is bonded to the oxygen of the cyclohexene ring.

21. A use as claimed is claim 19 where the compound is the compound recited in preceding claim 20.

## Patentansprüche

1. Verwendung einer Verbindung der Formel I 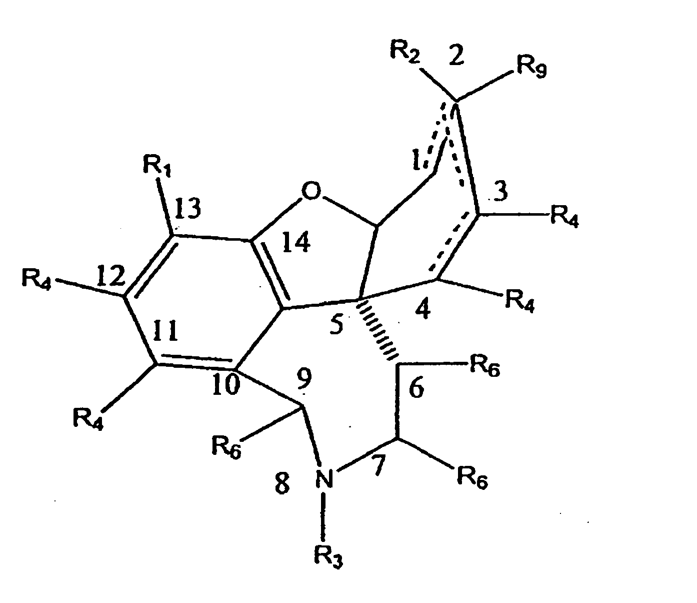 worin die unterbrochene Linie eine wahlweise vorhandene Doppelbindung in einem oder den beiden gezeigten Abschnitten darstellt;
R₁ und R₂ sind jeweils unabhängig ausgewählt aus der Gruppe, bestehend aus: Wasserstoff, Hydroxyl, Cyano, Sulfhydryl, Alkoxy mit 1 bis 6 Kohlenstoffatomen, Aryloxy, R₅-substituiertes Aryloxy, Aralkoxy, R₅-substituiertes Aralkoxy, Aryloxymethyl, R₅-substituiertes Aryloxymethyl, Alkanoyloxy, Hydroxy-substituiertes Alkanoyloxy, Benzoyloxy, R₅-substituiertes Benzoyloxy, Aryloxycarbonyl und R₅-substituiertes Aryloxycarbonyl;
R₃ ist Wasserstoff, geradkettiges oder verzweigtes Alkyl mit 1 bis 6 Kohlenstoffatomen, Cycloalkymethyl, Alkylphenyl, R₅-substituiertes Alkylphenyl, Heterocyclyl, das ausgewählt ist aus α- oder β-Furyl, α- oder β-Thienyl oder Thienyl, Pyridyl, Pyrazinyl und Pyrimidyl, Alkylheterocyclyl oder R'-substituiertes Heterocyclyl, worin Heterocyclyl wie vorstehend festgelegt ist; und R' ist Alkyl oder Alkoxy;
jedes R₄ ist unabhängig ausgewählt aus: Wasserstoff, Hydroxyl, Sulfhydryl, Alkyl, Aryl, Aralkyl, Alkoxy, Aryloxy, Alkanoyloxy, Nitro, Amino, N-Alkylamino, N-Arylamino, N-Alkarylamino, Fluor, Chlor, Brom und Iod;
R₅ ist ausgewählt aus den gleichen Gruppen wie R₄;
R₆ ist Wasserstoff;
R₉ ist Wasserstoff oder ist nicht vorhanden, wenn der Kohlenstoff in der 2-Stellung an einer Doppelbindung gebunden ist,
mit der Maßgabe, dass R₃ nicht Methyl ist, wenn R₁ Methoxy ist, R₂ ist Hydroxy und alle R₄ sind Wasserstoff, und mit der Maßgabe, dass die Verbindungen nicht solche sind, worin das Hydroxy von Galanthamin durch Methoxy ersetzt ist, durch Ethoxy, C₁- bis C₅-niederes Alkanyloxy, wie beispielsweise Acetyloxy, wobei die Methoxy-Gruppe ersetzt ist durch Wasserstoff, Methoxy, Ethoxy oder niederes Alkanyloxy mit 1 bis 5 Kohlenstoffatomen, wie beispielsweise Acetyloxy, und die Methyl-Gruppe substituiert an dem Stickstoffatom von Galanthamin ersetzt ist durch andere gradkettige oder verzweigte C₁- bis C₅-niedere AlkylGruppen, wie beispielsweise Ethyl, Cyclopropylmethyl oder Cyclobutylmethyl, Allyl, C₁- bis C₅-niederes Alkylphenyl oder substituiertes C₁- bis C₅-niederes Alkylphenyl, worin die Substituenten Fluor sind, Chlor, Brom, C₁- bis C₅-niederes Alkoxy, Hydroxy, Nitro, Amino, C₁- bis C₅-niederes Alkyl oder Acylamino mit 1 bis 5 Kohlenstoffatomen, Heteroaryl, C₁- bis C₅-niederes Alkyl, worin die Heteroaryl-Gruppe Thienyl ist, Furyl, Pyridyl, Pyrrolyl oder Pyrazinyl oder ein Cyano-Rest; oder unsubstituiertes und Halogen-substituiertes Benzoyl, C₁- bis C₅-niederes Alkyl, worin sich die Substituenten an dem Phenylring befinden, und Verbindungen, worin Wasserstoffatome in der "Kern"-Struktur ersetzt worden sind durch Fluor- oder Chlor-Gruppen;
oder ein pharmazeutisch akzeptables Säureanlagerungssalz davon für die Herstellung eines Medikaments zur Behandlung der Alzheimer Krankheit.

2. Verwendung nach Anspruch 1, worin die Verbindung, die verwendet wird, eine solche ist, worin R₁ und R₂ jeweils ausgewählt sind aus:
H, OR, SH, 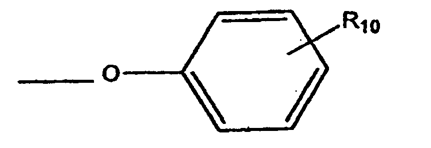 -OC(O)R, 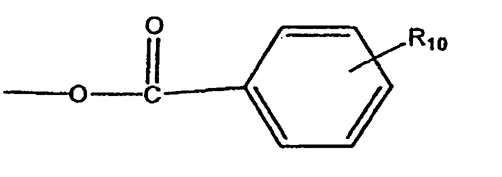 -OC(O)OR, 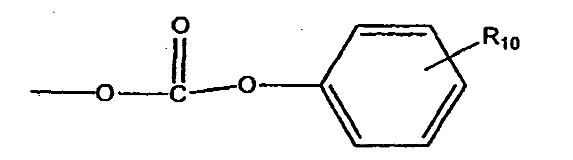 worin R Alkyl mit 1 bis 6 Kohlenstoffatomen ist oder Phenyl oder R₅-substituiertes Phenyl oder Benzyl oder R₅-substituiertes Benzyl, worin R₁₀ Wasserstoff ist, Alkyl oder Alkoxy, R₃ ist -H oder verzweigtes oder geradkettiges Alkyl oder 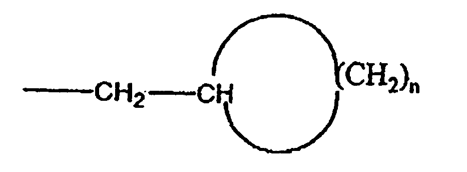 worin n 3, 4 oder 5 beträgt 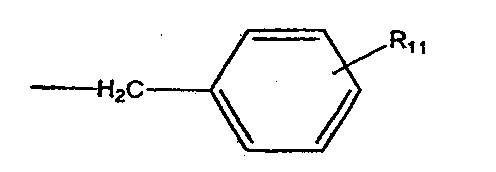 worin R₁₁ ist, wie vorstehend für R₁₀ festgelegt wurde 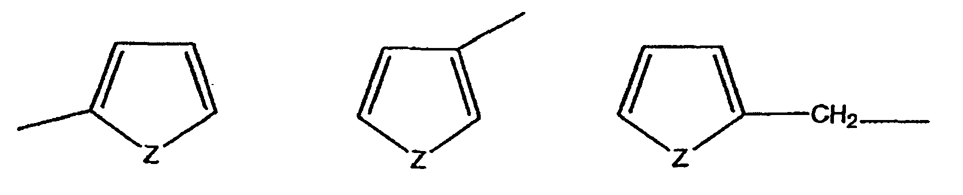 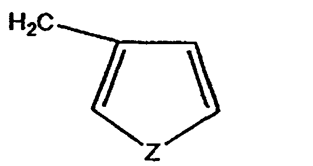 worin Z O, S oder NH ist.

3. Verwendung nach Anspruch 1, worin die Verbindung die Formel hat: 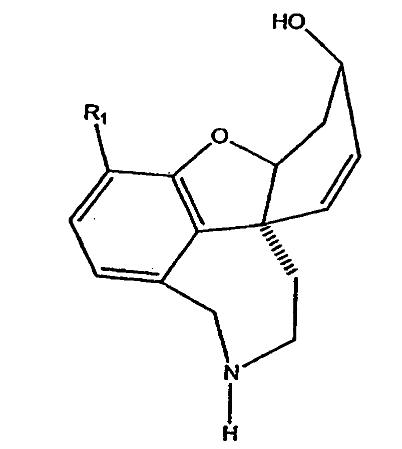 worin R₁ Hydroxy ist, C₁- bis C₆-niederes Alkoxy, Aryloxy, R₅-substituiertes Aryloxy.

4. Verwendung nach Anspruch 3, worin die Verbindung ausgewählt ist aus: O-Demethyl-, N-Demethyl-Galanthamin; O-Ethyl,O-Demethyl, N-Demethyl-Galanthamin; O-Phenyl-, O-Demethyl, N-Demethyl-Galanthamin; und O-Benzyl, O-Demethyl-Galanthamin.

5. Verwendung nach Anspruch 1, worin die Verbindung die Formel hat: 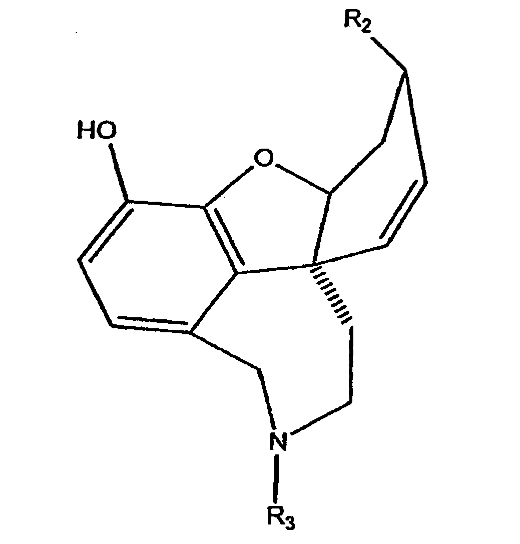 worin R₂ Hydroxy ist, C₁- bis C₆-Alkoxy, Aryloxy, und R³ Wasserstoff ist oder Alkyl mit 1 bis 6 Kohlenstoffatomen, wie beispielsweise Methyl oder Ethyl, Methylcyclopropyl oder Benzyl oder R⁵-substituiertes Benzyl.

6. Verwendung nach Anspruch 5, worin die Verbindung ausgewählt ist aus O-Demethyl-Galanthamin; O-Demethyl-Galanthamin; O-Ethyl-ether und das entsprechende N-Demethyl und N-Demethyl-N-Ethyl und N-Demethyl-N-Cyclopropylmethyl und N-Demethyl-N-Benzyl-Verbindungen.

7. Verwendung nach Anspruch 1, worin die Verbindung die Formel hat: 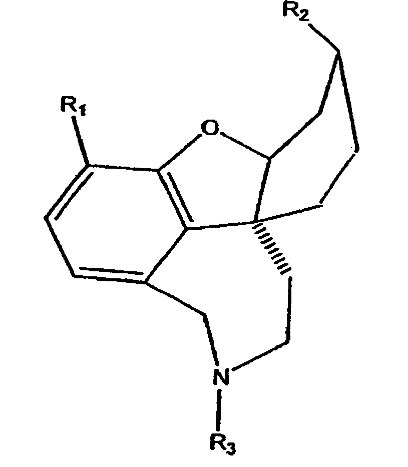 worin R₁, R₂ und R₃ wie vorstehend in Anspruch 1 festgelegt sind.

8. Verwendung nach Anspruch 7, worin die Verbindung, die eingesetzt wird, eine solche ist, worin R₁ Hydroxy oder C₁- bis C₆-Alkoxy ist, Benzyloxy oder R₅-substituiertes Benzyloxy, R₂ ist Hydroxy, C₁- bis C₆-Alkoxy, Aryloxy, Benzyloxy, und R₃ ist Wasserstoff, Methyl, Ethyl, Cyclopropylmethyl oder Benzyl.

9. Verwendung nach Anspruch 8, worin die Verbindung ausgewählt ist aus: O-Demethyl-lycoramin; N-Demethyl,O-Demethyl-lycoramin; N-Demethyl, N-Ethyllycoramin; N-Demethyl,N-Cyclopropylmethyl-lycoramin; N-Demethyl,N-Benzyl-lycoramin; O-Demethyl-lycoramin-ethylester; Deosxy-O-demethyl-lycoramin und--benzylether.

10. Verwendung nach Anspruch 1, worin die Verwendung die Formel hat: 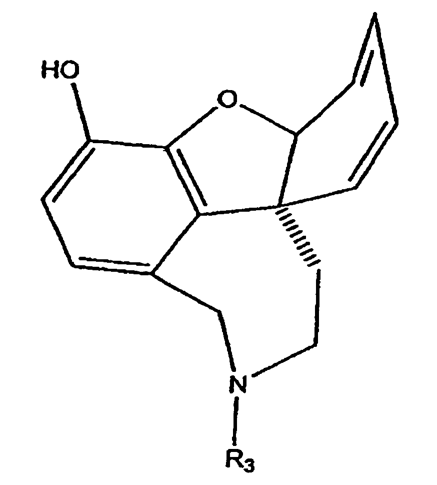 worin R₃ ausgewählt ist aus Wasserstoff, niederem Alkyl mit 1 bis 6 Kohlenstoffatomen, Cycloalkylmethyl oder Benzyl.

11. Verwendung nach einem der vorgenannten Ansprüche, worin die Verabreichung für eine parenterale Verabreichung mit einer Tagesdosis von 0,5 bis 1.000 mg einer Verbindung der Formel I nach Anspruch 1 oder eines pharmazeutisch akzeptablen Säureanlagerungssalzes davon ist.

12. Verwendung nach einem der vorgenannten Ansprüche, worin die Tagesdosisrate 50 bis 300 mg pro Tag beträgt.

13. Verwendung nach einem der vorgenannten Ansprüche 1 bis 10, worin das Medikament geeignet ist zur oralen Verabreichung im Bereich von 10 bis 2.000 mg pro Tag.

14. Verwendung nach Anspruch 13, wobei die Dosisrate 100 bis 600 mg pro Tag beträgt.

15. Verwendung nach einem der Ansprüche 1 bis 10, wobei das Medikament geeignet ist zur parenteralen Verabreichung in einer Dosisrate von 0,1 bis 4 mg/kg Körpergewicht eines Patienten.

16. Verwendung nach einem der Ansprüche 1 bis 10, wobei das Medikament geeignet ist für die Verabreichung intrazerebroventrikulär über ein implantiertes Reservoir mit einer Dosisrate von 0,01 bis 5,0 mg/kg pro Tag.

17. Verwendung nach einem der Ansprüche 1 bis 10, wobei das Medikament in Form einer Formulierung mit verzögerter Freisetzung vorliegt.

18. Verwendung einer Verbindung der Formel I 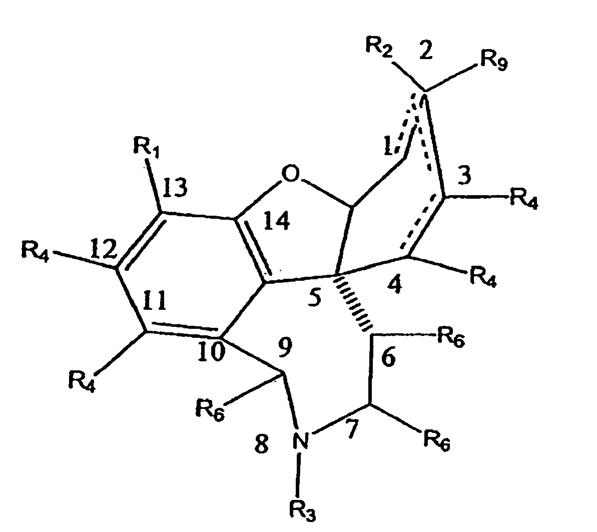 worin die unterbrochene Linie eine wahlweise vorhandene Doppelbindung in einem oder den beiden gezeigten Abschnitten darstellt,
mindestens ein R₁ und R₂ ist eine aliphatische oder Arylcarbamat-Gruppe, worin der aliphatische oder Aryl-Teil R₅ substituiert oder unsubstituiert sein kann,
und das andere der R₁ und R₂ ist unabhängig ausgewählt aus der Gruppe, bestehend aus: Wasserstoff, Hydroxyl, Cyano, Sulfhydryl, Alkoxy mit 1 bis 6 Kohlenstoffatomen, Aryloxy, R₅-substituiertem Aryloxy, Aralkoxy, R₅-substituiertem Aralkoxy, Aryloxymethyl, R₅-substituiertem Aryloxymethyl, Alkanoyloxy, hydroxy-substituiertem Alkyoyloxy, Benzyloxy, R₅-substituiertem Benzyloxy, Aryloxycarbonyl und R₅-substituiertem Aryloxycarbonyl, R₁ kann ebenfalls Alkyl mit bis zu 14 Kohlenstoffatomen sein oder Hydroxymethyl, R₂ kann ebenfalls Carboxymethyl mit der Maßgabe sein, dass mindestens eines von R₁ und R₂ Hydroxy ist;
R₃ ist Wasserstoff, geradkettiges oder verzweigtes Alkyl mit 1 bis 6 Kohlenstoffatomen, Cycloalkylmethyl, Phenyl, R₅-substituiertes Phenyl, Alkylphenyl, R₅-substituiertes Alkylphenyl, Heterocyclyl, ausgewählt aus α- oder β-Furyl, α- oder β-Thienyl oder Thienyl, Pyridyl, Pyrazinyl und Pyrimidyl, Alkyheterocyclyl oder R'-substituiertes Heterocyclyl, wobei Heterocyclyl wie vorstehend festgelegt ist und R'-Alkyl ist oder Alkoxy,
jedes R₄ ist unabhängig ausgewählt aus: Wasserstoff, Hydroxyl, Sulfhydryl, Alkyl, Aryl, Aralkyl, Alkoxy, Mercaptoalkyl, Aryloxy, Alkaryloxy, Nitro, Amino, N-Alkylamino, N-Arylamino, N-Alkarylamino, Fluor, Chlor, Brom, Iod,
R₅ ist ausgewählt aus den gleichen Gruppen wie R₄,
R₆ ist Wasserstoff,
R₉ ist Wasserstoff oder Alkyl mit 1 bis 6 Kohlenstoffatomen oder ist nicht vorhanden, wenn sich der Kohlenstoff in der 2-Stellung an einer Doppelbindung befindet,
oder ein pharmazeutisch akzeptables Säureanlagerungssalz davon, für die Herstellung eines Medikament für die Behandlung von Alzheimer Krankheit.

19. Verwendung nach Anspruch 18, wobei die Verbindung, die verwendet wird, eine solche ist, worin R₁ und R₂ jeweils ausgewählt sind aus:
H, OR, SH, 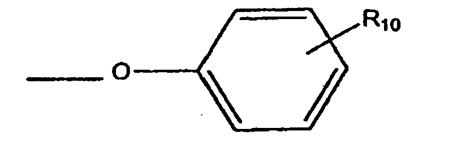 -OC(O)R, 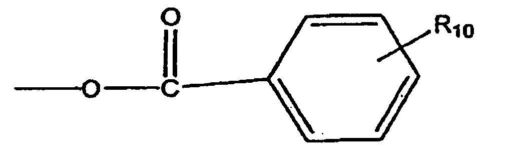 -OC(O)OR, 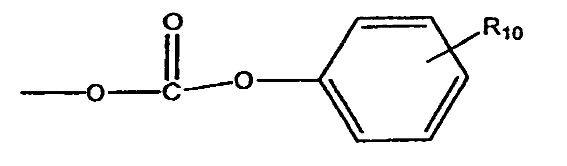 worin R Alkyl mit 1 bis 6 Kohlenstoffatomen ist oder Phenyl oder R₅-substituiertes Phenyl oder Benzyl oder R₅-substituiertes Benzyl, worin R₁₀ Wasserstoff ist, Alkyl oder Alkoxy, R₃ ist -H oder verzweigtes oder geradkettiges Alkyl oder 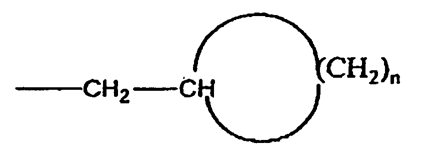 worin n 3, 4 oder 5 beträgt.

20. Verbindung, die ausgewählt ist aus O-Demethyl-Galanthamin-phenylcarbamat und O-Demethyl-N-Demethyl-Galanthamin, -Phenyl- und -Naphthylcarbamaten; O-Demethyl-Galanthamin-dimethylcarbamat und O-Dimethyl-Galanthamin-diethylcarbamat, worin die Carbamyl-Gruppe an dem Sauerstoff des Cyclohexen-Ringes gebunden ist.

21. Verwendung nach Anspruch 19, worin die Verbindung 19 die in Anspruch 20 genannte Verbindung ist.

## Revendications

1. Utilisation d'un composé de la formule (I) 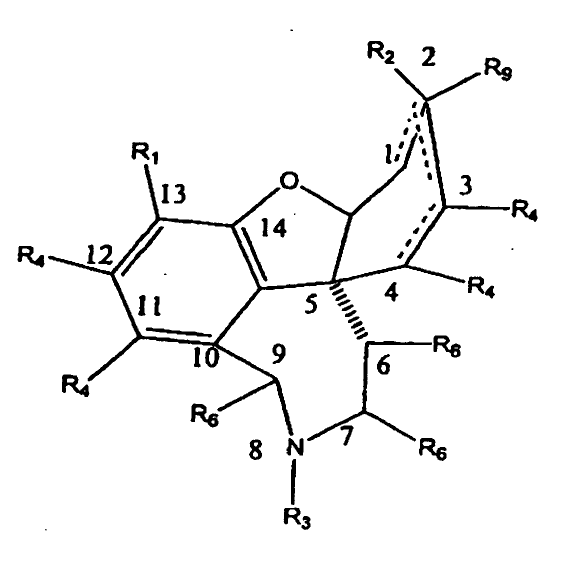 dans laquelle la ligne en pointillé représente une double liaison facultativement présente dans une ou dans les deux parties représentées,
R₁ et R₂ sont chacun choisis indépendamment parmi un atome d'hydrogène, un groupe hydroxyle, cyano, sulfhydryle, alcoxy de 1-6 atomes de carbone, aryloxy, aryloxy R₅-substitué, aralcoxy, aralcoxy R₅-substitué, aryloxyméthyle, aryloxyméthyle R₅-substitué, alcanoyloxy, alcanoyloxy hydroxy-substitué, benzoyloxy, benzoyloxy R₅-substitué, aryloxycarbonyle et aryloxycarbonyle R₅-substitué,
R₃ est un atome d'hydrogène, un groupe alkyle linéaire ou ramifié de 1-6 atomes de carbone, cycloalkylméthyle, alkylphényle, alkylphényle R₅-substitué, hétérocyclyle choisi parmi α- ou β-furyle, α- ou β-thiényle ou thiényle, pyridyle, pyrazinyle et pyrimidyle, alkyle hétérocyclyle ou hétérocyclyle R'-substitué, où hétérocyclyle est comme défini ci-dessus et R' est un groupe alkyle ou alcoxy,
chaque R₄ est indépendamment choisi parmi un atome d'hydrogène, un groupe hydroxyle, sulfhydryle, alkyle, aryle, aralkyle, alcoxy, aryloxy, alkaryloxy, nitro, amino, N-alkylamino, N-arylamino, N-alkarylamino, fluoro, chloro, bromo et iodo,
R₅ est choisi parmi les mêmes groupes que R₄,
R₆ est un atome d'hydrogène,
R₉ est un atome d'hydrogène ou est non existant lorsque le carbone en position 2 est fixé à une double liaison,
à condition que R₃ ne soit pas le groupe méthyle lorsque R₁ est le groupe méthoxy, R₂ est le groupe hydroxy et les R₄ sont un atome d'hydrogène et à condition que les composés ne sont pas ceux dans lesquels le groupe hydroxy de la galanthamine est remplacé par un groupe méthoxy, éthoxy, alcanoyloxy inférieur en C₁-C₅, tel que acétyloxy, le groupe méthoxy est remplacé par un atome d'hydrogène, un groupe méthoxy, éthoxy ou alcanoyloxy inférieur de 1 à 5 atomes de carbone, tel que acétyloxy, et le groupe méthyle substitué sur l'atome d'azote de la galanthamine est remplacé par d'autres groupes alkyles inférieurs en C₁-C₅ linéaires ou ramifiés, tels que éthyle, cyclopropylméthyle ou cyclobutylméthyle, allyle, alkylphényle inférieur en C₁-C₅ ou alkylphényle inférieur en C₁-C₅ substitué où les substituants sont fluoro, chloro, bromo, alcoxy inférieur en C₁-C₅, hydroxy, nitro, amino, alkyle inférieur en C₁-C₅ ou acylamino de 1 à 5 atomes de carbone, hétéroaryl(alkyle inférieur en C₁-C₅), où le groupe hétéroaryle est le groupe thiényle, furyle, pyridyle, pyrrolyle ou pirazinyle ou un radical cyano ; ou un groupe benzoyl(alkyle inférieur en C₁-C₅) non substitué ou substitué par un atome d'halogène, dans lequel les substituants se trouvent sur le cycle phényle et des composés dans lesquels les atomes d'hydrogène dans la structure de "noyau" ont été remplacés par des groupes fluoro ou chloro ;
ou un sel d'addition d'acide pharmaceutiquement acceptable de celui-ci pour la production d'un médicament destiné au traitement de la maladie d'Alzheimer.

2. Utilisation selon la revendication 1, dans laquelle le composé utilisé est un composé dans lequel R₁ et R₂ sont chacun choisis parmi H, OR, SH, 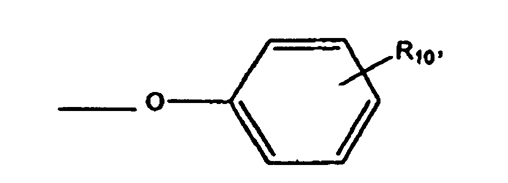 -OC(O)R, 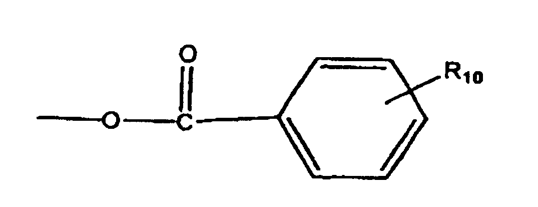 -OC(O)OR, 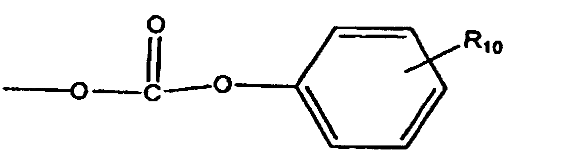 dans lesquelles R est un groupe alkyle de 1-6 atomes de carbone ou phényle, ou phényle R₅-substitué ou benzyle ou benzyle R₅-substitué, où R₁₀ est un atome d'hydrogène, un groupe alkyle ou alcoxy, R₃ est H ou un groupe alkyle linéaire ou ramifié ou 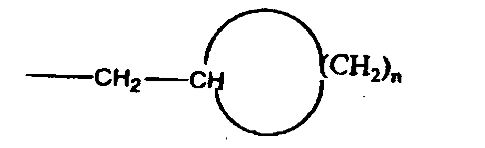 dans laquelle n est égal à 3, 4 ou 5 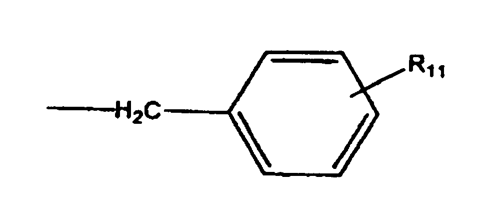 dans laquelle R₁₁ est comme défini pour R₁₀ ci-dessus. 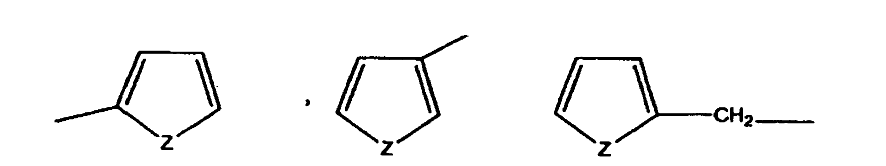 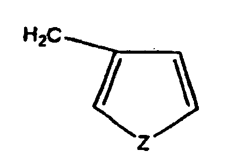 dans laquelle Z est O, S ou NH.

3. Utilisation selon la revendication 1, dans laquelle ledit composé est de la formule : 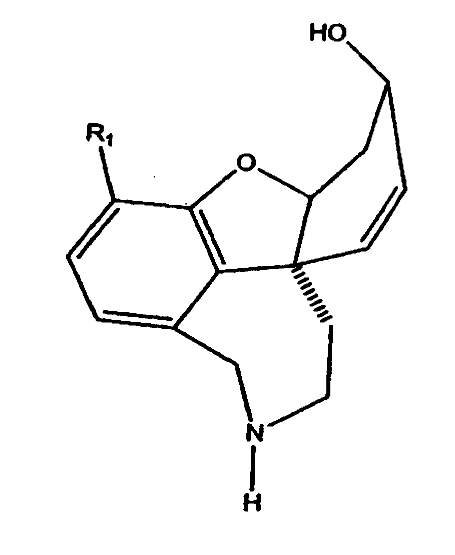 dans laquelle R₁ est un groupe hydroxy, alcoxy inférieur en C₁-C₆, aryloxy, aryloxy R₅-substitué.

4. Utilisation selon la revendication 3, dans laquelle ledit composé est choisi parmi l'O-deméthyl,N-deméthylgalanthamine, l'O-éthyl,O-deméthyl,N-deméthylgalanthamine ; l'O-phényl,O-eméthyl, N-deméthylgalanthamine ; et l'O-benzyl, O-deméthylgalanthamine.

5. Utilisation selon la revendication 1, dans laquelle ledit composé est de la formule : 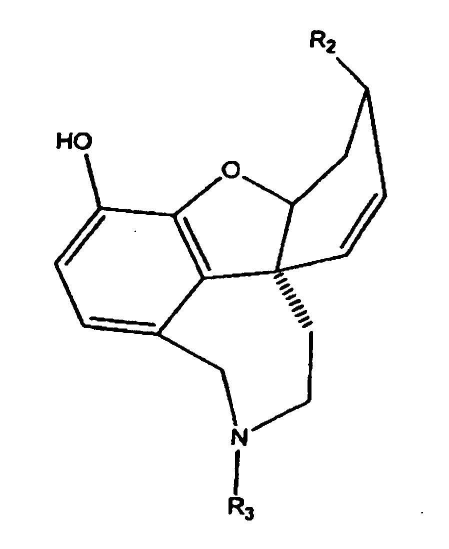 dans laquelle R₂ est un groupe hydroxy, alcoxy en C₁-C₆, aryloxy, et R₃ est un atome d'hydrogène ou un groupe alkyle de 1-6 atomes de carbone, tel que méthyle ou éthyle, méthylcyclopropyle ou benzyle ou benzyle R₅-substitué.

6. Utilisation selon la revendication 5, dans laquelle ledit composé est choisi parmi l'O-deméthylgalanthamine ; l'O-deméthylgalanthamine ; l'O-éthyléther ; et les composés correspondants de N-deméthyle et N-deméthyl-N-éthyle et N-deméthyl-N-cyclopropylméthyle et N-deméthyl-N-benzyle.

7. Utilisation selon la revendication 1, dans laquelle ledit composé est de la formule : 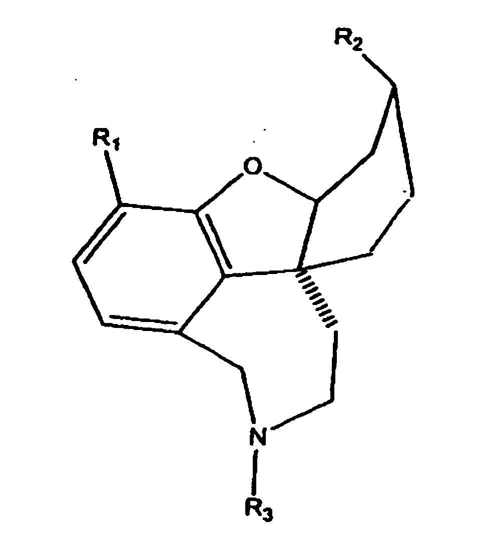 dans laquelle R₁ R₂ et R₃ sont comme définis dans la revendication 1.

8. Utilisation selon la revendication 7, dans laquelle le composé utilisé est un composé dans lequel R₁ est un groupe hydroxy, alcoxy en C₁-C₆, benzyloxy ou benzyloxy R₅-substitué, R₂ est un groupe hydroxy, alcoxy en C₁-C₆, aryloxy, benzyloxy et R₃ est un atome d'hydrogène, un groupe méthyléthyle, cyclopropylméthyle ou benzyle.

9. Utilisation selon la revendication 8, dans laquelle ledit composé est choisi parmi l'O-deméthyllycoramine ; la N-deméthyl,O-deméthyllycomarine ; la N-deméthyl-N-éthyllycoramine ; la N-deméthyl-N-cyclopropylméthyllycoramine, la N-deméthyl-N-benzyllycoramine ; l'O-deméthyllycoramine éthyléther ; la déoxy-O-deméthyllycoramine ; l'O-déoxydeméthyllycoramine et le benzyléther.

10. Utilisation selon la revendication 1, dans laquelle ledit composé est de la formule : 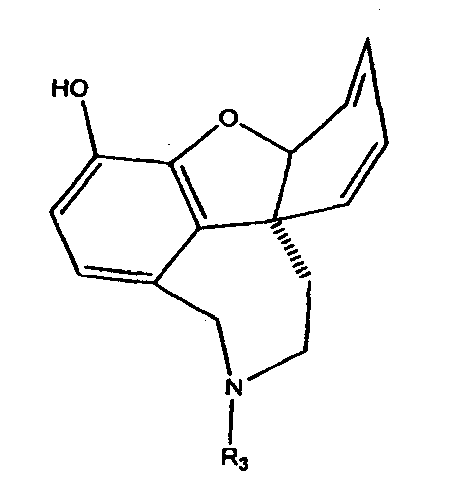 dans laquelle R₃ est choisi parmi un atome d'hydrogène, un groupe alkyle inférieur de 1 à 6 atomes de carbone, cycloalkylméthyle ou benzyle.

11. Utilisation selon l'une quelconque des revendications précédentes, dans laquelle l'administration est appropriée pour une administration parentérale à un dosage journalier de 0,5-1 000 mg d'un composé de la formule I comme revendiqué dans la revendication 1 ou comme un sel d'addition d'acide pharmaceutiquement acceptable de celui-ci.

12. Utilisation selon l'une quelconque des revendications précédentes, dans laquelle ledit taux de dosage est de 50-300 mg par jour.

13. Utilisation selon l'une quelconque des revendications précédentes 1-10, dans laquelle ledit médicament est approprié pour une administration orale et se trouve dans l'intervalle de 10-2 000 mg par jour.

14. Utilisation selon la revendication 13, dans laquelle ledit taux de dosage est de 100-600 mg par jour.

15. Utilisation selon l'une quelconque des revendications 1 à 10, dans laquelle ledit médicament est approprié pour une administration parentérale à un taux de dosage de 0,1 à 4 mg/kg de poids corporel d'un patient.

16. Utilisation selon l'une quelconque des revendications 1-10, dans laquelle ledit médicament est approprié pour une administration intracérébroventriculaire via un réservoir implanté à un taux de dosage de 0,01 à 5,0 mg/kg par jour.

17. Utilisation selon l'une quelconque des revendications 1-10, dans laquelle ledit médicament est dans la forme d'une formulation à libération prolongée.

18. Utilisation d'un composé de la formule 1 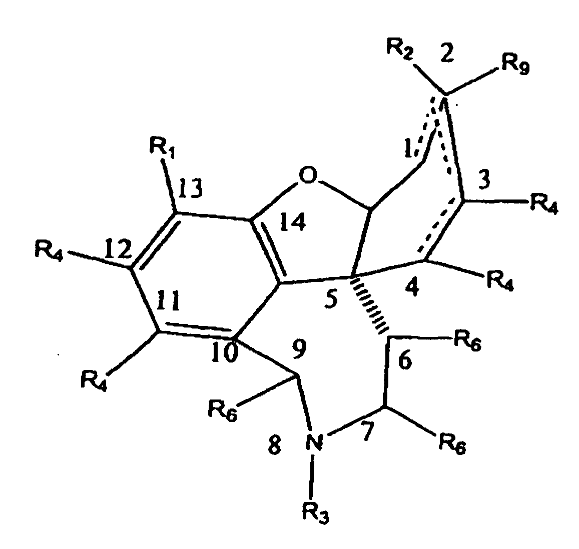 dans laquelle la ligne en pointillé représente une double liaison facultativement présente dans une ou dans les deux parties représentées,
au moins un parmi R₁ et R₂ est un groupe aliphatique ou arylcarbamate où la moitié aliphatique ou aryle peut être R₅-substitué ou non substitué,
et l'autre parmi R₁ et R₂ est choisi indépendamment parmi un atome d'hydrogène, un groupe hydroxyle, cyano, sulfhydryle, alcoxy de 1-6 atomes de carbone, aryloxy, aryloxy R₅-substitué, aralcoxy, aralcoxy R₅-substitué, aryloxyméthyle, aryloxyméthyle R₅-substitué, alcanoyloxy, alcanoyloxy hydroxy-substitué, benzoyloxy, benzoyloxy R₅-substitué, aryloxycarbonyle et aryloxycarbonyle R₅-substitué, R₁ peut également être un groupe alkyle avec jusqu'à 14 atomes de carbone ou hydroxyméthyle, R₂ peut également être un groupe carboxyméthyle à condition qu'au moins un parmi R₁ et R₂ soit un groupe hydroxy,
R₃ est un atome d'hydrogène, un groupe alkyle linéaire ou ramifié de 1-6 atomes de carbone, cycloalkylméthyle, phényle, phényle R₅-substitué, alkylphényle, alkylphényle R₅-substitué, hétérocyclyle choisi parmi α- ou β-furyle, α- ou β-thiényle ou thiényle, pyridyle, pirazinyle et pyrimidyle, alkyle hétérocyclyle ou hétérocyclyle R'-substitué, où hétérocyclyle est comme défini ci-dessus et R' est un groupe alkyle ou alcoxy,
chaque R₄ est indépendamment choisi parmi un atome d'hydrogène, un groupe hydroxyle, sulfhydryle, alkyle, aryle, aralkyle, alcoxy, mercaptoalkyle, aryloxy, alkaryloxy, nitro, amino, N-alkylamino, N-arylamino, N-alkarylamino, fluoro, chloro, bromo, iodo,
R₅ est choisi parmi les mêmes groupes que R₄,
R₆ est un atome d'hydrogène,
R₉ est un atome d'hydrogène ou un groupe alkyle de 1 à 6 atomes de carbone ou est non existant lorsque le carbone dans la position 2 est fixé à une double liaison,
ou un sel d'addition d'acide pharmaceutiquement acceptable de celui-ci pour la production d'un médicament destiné au traitement de la maladie d'Alzheimer.

19. Utilisation selon la revendication 18, dans laquelle le composé utilisé est un composé dans lequel R₁ et R₂ sont chacun choisi parmi H, OR, SH, 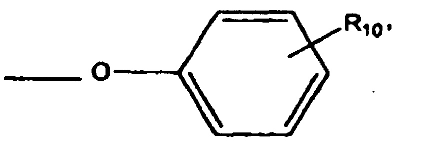 -OC(O)R, 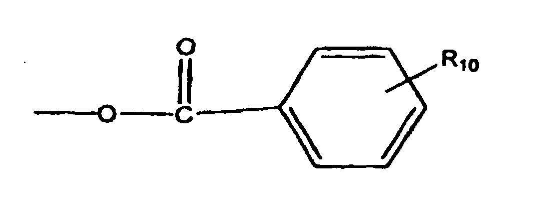 -OC(O)OR, 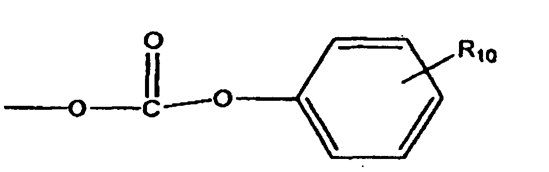 dans lesquelles R est un groupe alkyle de 1-6 atomes de carbone ou phényle ou phényle R₅-substitué ou benzyle, ou benzyle R₅-substitué, où R₁₀ est un atome d'hydrogène, un groupe alkyle ou alcoxy, R₃ est -H ou alkyle linéaire ou ramifié ou 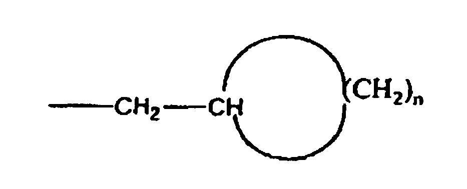 dans laquelle n est égal à 3, 4 ou 5.

20. Composé qui est choisi parmi l'O-deméthylgalanthamine-carbamate de phényle et les O-deméthyl-N-deméthylgalanthamine-carbamates de phényle et de naphtyle ; l'O-deméthylgalanthamine-carbamate de diméthyle; l'O-deméthylgalanthamine-carbamate de diéthyle, où le groupe carbamyle est lié à l'oxygène du cycle cyclohexène.

21. Utilisation selon la revendication 19, dans laquelle le composé est le composé donné dans la revendication précédente 20.
